# EUROPÄISCHE PATENTSCHRIFT

(11) **EP 1 302 175 B1**
(45) Veröffentlichungstag und Bekanntmachung des Hinweises auf die Patenterteilung: **04.07.2007**
(21) Anmeldenummer: 02018580.7
(22) Anmeldetag: 19.08.2002
(51) Int. Cl.: A61C 8/00, A61C 3/00, A61B 17/16

(54) **Implantat-Anordnung und Krone**
Implant arrangement and crown
Ensemble d'implant et couronne

(30) Priorität: 09.10.2001 CH 18642001
(43) Veröffentlichungstag der Anmeldung: 16.04.2003
(73) Patentinhaber: Hermann, Werner, 6312 Steinhausen (CH)
(72) Erfinder: Hermann, Werner, 6312 Steinhausen (CH); Schug, Jens, Dr.med.dent., 8458 Dorf (CH); Schmidlin, Patrick Roger, Dr.med.dent., 8057 Zürich (CH)
(74) Vertreter: OK pat AG

(56) Entgegenhaltungen:
- DE-A- 19 949 385
- US-A- 1 604 695
- US-B1- 6 270 346
- US-B1- 6 280 191
- US-B1- 6 309 220

## Beschreibung

Die Erfindung betrifft eine Implantat-Anordnung nach dem Oberbegriff des Anspruchs **1** und eine Krone nach Anspruch **20**.

Zahnersatzteile mit Implantat-Anordnungen dieser Art sind auch unter der Bezeichnung Zahnimplantate bekannt; diese Bezeichnung ist ungenau, da nicht jeder Bestandteil eines solchen sogenannten Zahnimplantates tatsächlich direkt implantiert ist; implantiert ist nur ein Bestandteil, nämlich der proximalste, auf welchem die restlichen Bestandteile direkt oder indirekt befestigt sind.

Herkömmliche Zahnersatzteile bestehen meist aus einer Krone, welche im Wesentlichen den sichtbaren Teil des Zahnersatzteiles bildet, und aus einer Basis mit einer Kronenaufnahme, an welcher die Krone befestigt wird. Die Basis wird im Allgemeinen durch eine Implantat-Anordnung und ein Adapterteil, das gelegentlich auch als Abutment bezeichnet wird, gebildet. Die Implantat-Anordnung ist der einzige im wahren Sinn des Wortes implantierte Bestandteil des Zahnersatzteiles. Die Implantat-Anordnung wird im Kieferknochen eines Patienten implantiert, meist eingeschraubt. Das Adapterteil wird am distalen Ende der Implantat-Anordnung befestigt und weist die Kronenaufnahme auf. Die an der Kronenaufnahme des Adapterteils befestigte Krone kann ein- oder mehrteilig sein; beispielsweise kann sie aus einem sogenannten Gerüst und einer Verblendung bestehen. Ein Zahnersatzteil dieser Art dient zum Ersatz eines einzigen Zahnes. Es sind aber auch Zahnersatzteile bekannt, mit welchen mehrere benachbarte Zähne ersetzt werden können. Solche Zahnersatzteile umfassen im Allgemeinen, aber nicht notwendigerweise, eine zweite Basis, und die Krone ist als Brücke oder Kronenblock ausgebildet.

Bis ein herkömmliches Zahnersatzteil funktionstüchtig montiert ist, sind viele Schritte notwendig.

Als Erstes muss meist ein Knochenaufbau stattfinden. Viele Zahnersatzteile werden für Patienten benötigt, die nicht nur schlechte Zähne besitzen sondern auch unter Zahnfleisch- und Kieferknochenschwund leiden. Dies trifft häufig auf ältere Patienten zu. Jüngere Patienten beziehungsweise Patienten ohne Zahnfleisch- und Kieferknochenschwund benötigen Zahnersatzteile meist nur nach Unfällen; hierbei kann allerdings der Kieferknochen ebenfalls traumatisiert sein. Liegt ein Kieferknochendefizit vor, so muss herkömmlicherweise zuerst ein Knochenaufbau vorgenommen werden. Hierzu wird vor der Implantierung der Implantat-Anordnung entweder eine künstliche Ersatzmasse am Kieferknochen angebracht, was mit der Erzeugung eines Operationstraumas im Kieferbereich verbunden ist, oder es wird natürliche Knochenmasse, beispielsweise aus dem benachbarten Kieferbereich oder aus dem Beckenbereich, implantiert, was mit der Erzeugung von zwei Operationstraumata, im Beckenbereich und im Kieferbereich, verbunden ist. Gerade ältere Patienten, die am häufigsten ein Zahnersatzteil dieser Art benötigen, ertragen im Allgemeinen Operationen schlecht. Und nach erfolgtem Knochenaufbau muss noch die Implantat-Anordnung im aufgebauten Kieferknochen befestigt werden, was ein weiteres Operationstrauma zur Folge hat, wobei jedes Operationstrauma nicht nur Schmerzen verursacht, sondern auch die Gefahr grösserer Infektionen mit sich bringt.

Steht genügend Knochenmasse zur Verfügung, sei es von Natur aus oder nach einem Knochenaufbau, so findet wie schon erwähnt das Implantieren der üblicherweise als Implantatpfosten bezeichneten Implant-Anordnung statt. Der Implantatpfosten benötigt eine gewisse Zeitspanne, um in erforderlicher Weise im Kieferknochen einzuwachsen oder, besser gesagt, von Kieferknochen umwachsen zu werden. Erst dann kann das Adapterteil befestigt werden.

Es muss festgestellt werden, dass die herkömmlichen Zahnersatzteile, obwohl sie sich im Gebrauch nicht schlecht bewähren, mit vielen Nachteilen verbunden sind, und zwar wegen des Montageprozesses, der auch den Knochenaufbau einschliesst und im Allgemeinen langwierig, zeitraubend, schmerzhaft und mit Infektionsrisiken verbunden ist.

Es ist deshalb **Aufgabe** der Erfindung
- eine Implantat-Anordnung für ein Zahnersatzteil zu schaffen, das den Montageprozess erleichtert und besonders geeignet ist in Fällen, in denen das Implantieren in einen durch Knochenmangel beeinträchtigten Kieferknochen erfolgen muss;
- eine zur Befestigung auf dieser Implantat-Anordnung geeignete Krone vorzuschlagen, welche die Implantat-Anordnung stabilisiert;

Ein Zahnersatzteil kann beispielsweise eine herkömmliche Implantat-Anordnung aufweisen, wie sie aus der US-6, 270,346 bekannt ist. Diese weist ein proximales Implantat-Element zur Befestigen in einem proximalen Kieferknochenbereich und ein distales Implantat-Element zur Befestigung im angrenzenden distalen Kieferknochenbereich auf. Eine Schraubenvorrichtung dient dazu, die beiden Kieferbereiche nach Trennung voneinander zu beabstanden, um in der entstehenden Lücke die Neubildung von Knochenmaterial zu veranlassen.

Die **Lösung** dieser Aufgabe erfolgt
- für die Implantat-Anordnung der eingangs genannten Art durch die Merkmale des kennzeichnenden Teils des unabhängigen Anspruchs **1;** und
- für die Krone der eingangs genannten Art durch die Merkmale des unabhängigen Anspruchs **20**.

Bevorzugte Weiterbildungen der erfindungsgemässen Gegenstände sind durch die jeweiligen abhängigen Ansprüche definiert.

Es sei hier darauf hingewiesen, dass die Bezeichnungen 'distal' und 'proximal' in der vorliegenden Beschreibung im gleichen Sinne wie in der Allgemeinmedizin verwendet werden; 'distal' und ,proximal` sind relative Begriffe, wobei mit 'distal' körperzentrumsfernere und mit 'proximal' körperzentrumsnähere Teile bezeichnet werden. Die Begriffe 'distal' und 'proximal' hier nicht nur für Körperteile sondern auch für die Zahnersatzteile benutzt, und sie werden auch für die noch nicht implantierten definitiven und temporären Bestandteile der Zahnersatzteile benutzt, wobei sich 'distal' und 'proximal' auf die Lagen beziehen, die die Bestandteile im montierten Zustand im Kiefer der Patienten definitiv oder temporär einnehmen. Schliesslich werden die Begriffe 'proximal' und 'distal' auch für die In strumente beziehungsweise deren Anordnung im Gebrauch benutzt, mit deren Hilfe das neue Implantat-Element montiert werden kann.

Der Grundgedanke, der zur vorliegenden Erfindung führte, besteht darin, den zur Aufnahme der Implantat-Anordnung bestimmten, aber durch Knochenschwund reduzierten Kieferknochen nicht mittels künstlichen Knochenmaterials oder echten, aber transplantierten Knochenmaterials aufzubauen, sondern den an sich vorhandenen, aber reduzierten Kieferknochen zu einer Knochenbildung anzuregen. Diese Anregung zur Knochenbildung soll nicht durch zusätzliche Massnahmen oder Vorrichtungen sondern durch die Implantat-Anordnung selbst erfolgen. Zu diesem Zwecke weist die erfindungsgemässe Implantat-Anordnung ein erstes, nach dem Implantieren proximales Implantat-Element und ein zweites, nach dem Implantieren distales Implantat-Element auf. Das proximale Implantat-Element wird in einem proximalen Kieferknochenbereich befestigt beziehungsweise tief eingeschraubt. Das distale Implantat-Element wird, im Wesentlichen koaxial und benachbart zum proximalen Implantat-Element, in einem distalen Kieferknochenbereich befestigt beziehungsweise weniger tief eingeschraubt als das proximale Implantat-Element. Nachdem die beiden Implantat-Elemente implantiert sind, wird der Kieferknochen zwischen dem proximalen und dem distalen Kieferknochenbereich mindestens teilweise getrennt beziehungsweise angeritzt. Hierfür werden besondere Massnahmen getroffen, die weiter unten ausführlich beschrieben sind. Anschliessend erfolgt durch eine Manipulation der Implantat-Elemente eine Verschiebung des distalen Kieferknochenbereiches in distaler Richtung vom proximalen Kieferknochenbereich weg; dieser Vorgang wird als Distraktion bezeichnet. Die Distraktion geschieht unter weiterer Zertrennung des schon teilweise getrennten oder angeritzten Kieferknochens. Bei der Distraktion des distalen Kieferknochenbereiches erweitert sich die Trennfuge zwischen dem proximalen und dem distalen Kieferknochenbereich zu einem sichtbaren Spalt. Dieser Spalt füllt sich nach und nach mit natürlichem, sich neu bildendem Knochenmaterial. Das zusätzliche Knochenmaterial für einen Knochenaufbau, welches bei einem herkömmlichen künstlichen, das heisst operativ durchgeführten Knochenaufbau am distalen Ende des Kieferknochens angelagert wurde, wird mit dem neuen Verfahren im Spalt in natürlicher Weise zwischen dem proximalen und dem distalen Kieferbereich angelagert. Es ist jedem Fachmann bekannt, dass die Bildung von Knochenmaterial in Spalten besonders gut vor sich geht, und dass gewisse Spaltweiten und ein gewisses Mass an mechanischer Zugbeanspruchung für die Bildung von Knochenmaterial optimal sind; die Spaltweite zwischen dem proximalen und dem distalen Kieferknochenbereich und wenn möglich auch die Beanspruchung werden entsprechend gewählt. Während also herkömmlicherweise der Knochenschwund durch eine künstliche Masse oder durch transplantiertes Knochenmaterial auf dem Rest des Kieferknochens kompensiert werden musste, wird bei der Verwendung der Basis nach der Erfindung der Knochenschwund durch natürliches, an Ort gewachsenes Knochenmaterial im Spalt zwischen dem proximalen und dem distalen Kieferknochenbereich kompensiert.

Gegenüber einer herkömmlichen Basis für ein Zahnersatzteil weist die neue Implantat-Anordnung zahlreiche Vorteile auf. Der Wesentlichste dieser Vorteile liegt darin, dass insgesamt nur ein invasiver Eingriff notwendig ist, so dass alle Gefahren und Unannehmlichkeiten von Operationen stark reduziert werden. Das Zahnersatzteil ist insgesamt in kürzerer Zeit fertig montiert. Der Knochenaufbau geschieht auf natürliche Weise und gewissermassen in situ.

Vorzugsweise bilden das gesamte proximale Implantat-Element und das gesamte distale Implantat-Element definitive Bestandteile des zu erstellenden Zahnersatzteiles.

Zur Distraktion des distalen Kieferknochenbereiches in distaler Richtung unter Zugbeanspruchung werden Mittel benutzt, die vorzugsweise ebenfalls Bestandteile der Implantat-Anordnung sind und die hierbei die Funktion von Werkzeugen übernehmen.

Um das Gesamtvolumen der definitiv im Kieferbereich des Patienten verbleibenden Implantat-Anordnung möglichst gering zu halten, ist es vorteilhaft, die Mittel zur Verschiebung des distalen Implantat-Elementes vor dem Anbringen der Krone mindestens teilweise zu entfernen; diese Mittel bilden somit nur temporäre Bestandteile der Implantat-Anordnung nach der Erfindung.

Die Mittel zur Verschiebung des distalen Kieferknochenbereiches durch Manipulation des proximalen und des distalen Implantat-Elementes bestehen aus mindestens einem Hilfselement der Implantat-Anordnung. Dieses Hilfselement wird mit dem distalen Implantat-Element verbunden, vorzugsweise mittels einer Schraubverbindung.

In einer ersten Ausbildungsform der Implantat-Anordnung nach der Erfindung erfüllt das Hilfselement die Funktionen eines Zuggliedes und eines Stellgliedes. Hierzu wird das Hilfselement mit dem distalen Ende des distalen Implantat-Elementes so weit wie möglich verschraubt. Bei einer Fortsetzung der Drehung des Hilfselementes würde dann das distale Implantat-Element mitgenommen und ebenfalls tiefer eingeschraubt. Das distale Implantat-Element ist aber bereits so tief wie möglich in den distalen Kieferknochenbereich eingeschraubt, nämlich bis zum Anschlag am proximalen Implantat-Element, und kann durch weitere Drehung nicht tiefer eingeschraubt werden. Die weitere Drehung des Hilfselementes bewirkt daher die Distraktion des distalen Kieferknochenbereiches in distaler Richtung, oder, in anderen Worten ausgedrückt, die Verschiebung des distalen Kieferknochenbereiches vom proximalen Kieferknochenbereich weg. Dadurch entsteht, wie oben beschrieben, eine Trennfuge und aus der Trennfuge ein Spalt zwischen dem proximalen und dem distalen Kieferknochenbereich. Die durch die Distraktion wirkenden Kräfte regen die natürliche Bildung von Knochenmaterial an, so dass sich dieser Spalt nach und nach mit Knochenmaterial füllt.

Das Hilfselement weist im Allgemeinen einen proximalen Gewindeabschnitt auf, der mit einem distalen Gewindeabschnitt des distalen Implantat-Elementes verschraubt wird. Es hat sich als besonders günstig erwiesen, das Hilfselement hülsenartig auszubilden, so dass sein erwähnter Gewindeabschnitt ein Innengewinde enthält, während das komplementäre Gewinde des distalen Implantat-Elementes ein Aussengewinde ist. Die umgekehrte Anordnung der Gewinde oder eine andere Verbindungsart ist auch möglich.

In einer zweiten Ausbildung der Implantat-Anordnung nach der Erfindung umfassen die Mittel zum Verschieben des distalen Implantat-Elementes das erwähnte Hilfselement und ein weiteres Hilfselement, wobei das erste Hilfselement die Funktion eines Zuggliedes und das weitere Hilfselement die Funktion eines Stellgliedes hat. Die Hilfselemente sind in Richtung der Implantat-Längsachse relativ zueinander verschiebbar und bewegungsmässig mit den Implantat-Elementen gekoppelt, derart, dass das distale Implantat-Element mit dem ersten Hilfselement und das proximale Implantat-Element mit dem weiteren Hilfselement solidarisch bewegbar ist. Das weitere, als Stellglied ausgebildete Hilfselement ist so verstellbar, dass bei seiner Verstellung eine Relativverschiebung zwischen den Hilfselementen in Richtung der Implantat-Längsachse stattfindet. Diese Relativverschiebung der Hilfselemente bewirkt eine gleichzeitige Relativverschiebung der Implantat-Elemente. Weil das proximale Implantat-Element im Wesentlichen ortsfest im proximalen Kieferknochenbereich verankert und nicht bewegbar ist, manifestiert sich die Relativverschiebung der Implantat-Elemente in einer Distraktion des distalen Implantat-Elementes in distaler Richtung.

Auch bei dieser zweiten Ausbildung der Implantat-Anordnung kann das Hilfselement, das als Zugglied benutzt wird, hülsenartig, das heisst als Zughülse, ausgebildet sein. Das zweite Hilfselement, das als Stellglied benutzt, kann als Stellschraube ausgebildet sein. Zughülse und Stellschraube sind miteinander so verbunden, dass sie eine relative Verschiebung in Richtung der Implantat-Längsachse durchführen können. Das weitere Hilfselement, das als Stellglied ausgebildet ist, kann ein Gewinde und eine Wirkfläche aufweisen; dieses Gewinde des weiteren Hilfselementes ist mit einem komplementären Gewinde des erstgenannten Hilfselementes verstellbar verschraubt, so dass eine Gewindeverbindung gebildet wird; die Wirkfläche des Stellgliedes, das heisst des weiteren Hilfselementes, liegt an einer Druckfläche des proximalen Implantat-Elementes an. Zur Betätigung des Stellgliedes wird dieses gedreht, das heisst die Gewindeverbindung wird angezogen; die Wirkfläche des Stellgliedes stützt sich hierbei an dieser Druckfläche ab und wird auf die Druckfläche des proximalen Implantat-Elementes vorgespannt. Das Anziehen der Gewindeverbindung hat daher die Folge, dass das erste Hilfselement, das heisst das Zugglied, sich in distaler Richtung verschiebt und hierbei das distale Implantat-Element mitnimmt, wobei das letztere seinerseits den distalen Kieferknochenbereich mitnimmt und vom proximalen Kieferknochenbereich entfernt. Dadurch entsteht, in gleicher Weise wie oben beschrieben, ein Spalt zwischen dem proximalen Kieferknochenbereich und dem distalen Kieferknochenbereich, der sich nach und nach mit Knochenmaterial füllt. Nach Beendigung der Distraktion wird das distale Implantat-Element mit Vorteil wieder soweit eingeschraubt, dass es am proximalen Implantat-Element anliegt, da dadurch die beiden definitiven Bestandteile der Implantat-Anordung in eine stabilere Lage gebracht werden.

Es wurde weiter oben beschrieben, dass Zahnersatzteile aus einer Implantat-Anordnung, einer Kronenaufnahme und einer Krone bestehen. Die Kronenaufnahme ist herkömmlicherweise an einem Adapterteil gebildet, das im montierten Zustand des Zahnersatzteiles zwischen der Krone und der Implantat-Anordnung angeordnet ist. In einer besonders vorteilhaften Ausbildung der Erfindung ist nun die neue Implantat-Anordnung so ausgebildet, dass sich das Adapterteil erübrigt. Zu diesem Zwecke wird an einem der Implantat-Elemente eine integrale Kronenaufnahme gebildet, so dass die Krone direkt am Implantat-Element befestigt werden kann.

Je nach der Ausbildung der Implantat-Anordnung kann die Kronenaufnahme entweder am proximalen oder am distalen Implantat-Element angeordnet sein. Eine Kronenaufnahme am proximalen Implantat-Element kann zu einer stabileren Fixierung der Krone Befestigung führen. Die Montage der Krone wird erleichtert, wenn die Kronenaufnahme am distalsten Bereich der Implantat-Anordnung vorgesehen ist; dies ist aber nicht zwingend, denn eine Krone mit einer geeigneten, genügend grossen Ausnehmung muss nicht am distalsten Bereich der Implantat-Anordnung befestigt sein. Bei einer in vielen Beziehungen optimalen Ausbildung weist das proximale Implantat-Element einen Ansatz auf, dessen distales Ende das distale Implantat-Element in distaler Richtung überragt und als Kronenaufnahme ausgebildet ist. Mit dieser Anordnung erreicht man sowohl eine stabile Befestigung der Implantat-Anordnung im Kieferknochen als auch eine einfache Montage der Krone.

Besonders günstig ist es, die Kronenaufnahme mit einem Aussengewinde zu versehen, so dass eine, ein komplementäres Innengewinde aufweisende, Krone auf den Ansatz aufgeschraubt werden kann.

Da Mikrobewegungen zwischen den verschiedenen definitiven Bestandteilen eines Zahnersatzteiles zu Verletzungen des angrenzenden lebenden Kieferbereiches führen können, werden diese Bestandteile vorzugsweise so ausgebildet und angeordnet, dass sie im montierten Zustand fest zusammengefügt beziehungsweise zusammengespannt sind. Definitive Bestandteile eines Zahnersatzteiles sind mindestens das proximale Implantat-Element, das distale Implantat-Element und die Krone.

Eine zusammengespannte Anordnung der definitiven Bestandteile des Zahnersatzteiles erhält man, indem man ein Spannglied auf einen Ansatz des proximalen Implantat-Elementes befestigt, vorzugsweise aufschraubt, und hierbei das distale Implantat-Element zwischen dem Spannglied und dem proximalen Implantat-Element einspannt. Beim Anziehen der Schraubverbindung, durch welche das Spannglied am proximalen Implantat-Element befestigt wird, übt das Spannglied eine Zugkraft auf das proximale Implantat-Element und eine Druckkraft auf das am proximalen Implantat-Element anliegende distale Implantat-Element aus.

Das fertig montierte Spannglied wird anschliessend durch die Krone überdeckt, das heisst, es befindet sich im montierten Zustand des Zahnersatzteils in einer Ausnehmung der Krone. Die Krone kann beispielsweise durch Kleben oder Zementieren befestigt werden. Hierbei kann die Krone auch aus einem Material hergestellt sein, das sich nicht für eine Schraubverbindung eignet.

Bei einer besonders rationellen Ausbildung übernimmt die Krone auch die Funktion des Spanngliedes; hierzu weist sie ein Innengewinde auf, mit welchem sie auf das proximale Implantat-Element aufgeschraubt wird.

Eines der Probleme, die im Zusammenhang mit Implantat-Anordnungen gelöst werden müssen, besteht darin, den Zutritt von Infektionserregern zu empfindlichen Stellen zu verhindern. Um Infektionen durch eindringende Bakterien zu verhindern, ist daher anzustreben, dass beide Implantat-Elemente und die Mittel zum Verschieben des distalen Implantat-Elementes so ausgebildet sind, dass die Implantat-Anordnung dichtend am Kieferknochen und im Zahnfleisch angeordnet werden kann, um den Zutritt von Mikroorganismen oder Fremdkörpern zu verhindern. Zu diesem Zwecke können die Mittel zum Verschieben des distalen Kieferknochenbereiches so ausgebildet sein, dass sie dichtend wirken. Beispielsweise kann das als Zughülse ausgebildete Hilfselement eine zulaufende Fläche besitzen, die im montierten Zustand satt an einer komplementären konischen Fläche des distalen Implantat-Elementes zur Anlage kommt. Im weiteren weist das Hilfselement in seinem distalen Endbereich vorzugsweise einen dichtenden Abschluss auf. Ist nur ein einziges Hilfselement vorhanden, das als Zug- und als Stellglied wirkt, so kann dafür beispielsweise eine am distalen Ende verschlossene Zughülse benutzt werden. Sind zwei Hilfselemente, nämlich ein Zugglied in Form einer Zughülse und ein Stellglied in Form einer Stellschraube vorhanden, so wird vorzugsweise ein Zwischenelement vorgesehen, das durch ein geeignetes, beispielsweise elastisches, Dichtungselement gebildet ist unddie Verbindungsstelle zwischen Zughülse und Stellschraube abdichtet.

Die Implantat-Elemente sind, wie weiter oben erwähnt, vorzugsweise so beschaffen, dass ihre Montage im entsprechenden Kieferknochenbereich durch Einschrauben erfolgt. Hierzu weisen sie einen Abschnitt mit einem Aussengewinde auf. Ein besonders geeignetes Aussengewinde ist, wie schon erwähnt, in der EP-1 112 722-A2 beschrieben. Es handelt sich dabei um ein selbstschneidendes Gewinde. Dies hat den Vorteil, dass die Vorgänge des Vorbohrens und des Gewindeschneidens entfallen und keine Zentrierungsprobleme beim Einschrauben des Implantat-Elementes entstehen.

Die Erfindung befasst sich nicht nur mit der oben beschriebenen zweiteiligen Implantat-Anordnung sondern auch mit zwei Instrumenten, welche zur Montage der Implantat-Anordnung benutzt werden und diese Montage wesentlich erleichtern.

Hierbei handelt es sich um ein Spreizinstrument und um ein Kerb- beziehungsweise Trenninstrument.

Ein wesentlicher Vorteil der neuen Implantat-Anordnung, auf den schon weiter oben hingewiesen wurde, ist darin zu sehen, dass zur Montage eines Zahnersatzteiles nur eine einzige invasive Handlung stattfinden muss, nämlich das Implantieren der Implantat-Anordnung. Hierbei wird in herkömmlicher Weise das proximale Implantat-Element im noch vorhandenen oder zuvor aufgebauten Kieferknochen implantiert. Dieses proximale Implantat-Element wird, vorzugsweise durch Einschrauben, so tief in einem proximalen Kieferknochenbereich befestigt, dass sein proximales Ende im Allgemeinen in den Wurzelkanal desjenigen Zahnes zu liegen kommt, der durch ein Zahnersatzteil ersetzt werden muss. Es wird hier davon ausgegangen, dass bei der verwendeten Implantat-Anordnung das distale Ende des proximalen Implantat-Elementes nach dem Implantieren aus dem Kieferknochen und dem angrenzenden Zahnfleisch ins Freie ragt. Anschliessend an das Implantieren des proximalen Implantat-Elementes wird das distale Implantat-Element implantiert, und zwar angrenzend an das proximale Implantat-Element in einem distalen Kieferknochenbereich. Danach werden die Mittel zum Distrahieren des distalen Kieferknochenbereiches befestigt. Hierzu können, wie weiter oben erwähnt, spezielle Massnahmen getroffen werden, für welche die im folgenden beschriebenen Instrumente, nämlich ein Spreizinstrument und ein Kerb- beziehungsweise Trenninstrument benutzt werden, die im Folgenden beschrieben werden.

Ein Spreizinstrument, insbesondere das nun beschriebene Spreizinstrument, wird eingesetzt, um den Kieferknochen über einen Höhenbereich, in der er eine geringe Wandstärke aufweist, aufzuspreizen. Dieser Höhenbereich entspricht im Allgemeinen etwa dem distalen Kieferknochenbereich. Das Aufspreizen des distalen Kieferknochenbereichs erfolgt vorzugsweise nach der Implantation des proximalen Implantat-Elementes, aber vor der Implantation des distalen Implantat-Elementes. Zur Vorbereitung der Implantation wird das Zahnfleisch in der entsprechenden Höhe chirurgisch geöffnet und vom Kieferknochen weggehalten. Dann wird das proximale Implantat-Element montiert beziehungsweise eingeschraubt. Hierfür ist im Allgemeinen eine Vorbohrung beziehungsweise Aushöhlung des Kieferknochens notwendig, doch weist diese nur einen geringen Durchmesser auf. Danach erfolgt das oben erwähnte Aufspreizen des Kieferknochens, wobei die Aushöhlung um das proximale Implantat-Element aufgespreizt und damit ein Freiraum für das Einbringen des distalen Implantat-Elementes geschaffen wird. Dank des Aufspreizens ist es nicht notwendig, im Höhenbereich der Aufspreizung, wo wenig Knochenmaterial vorhanden ist, eine Vorbohrung mit einem relativ grossen Durchmesser anzubringen, in welche das distale Implantat-Element eingeschraubt werden kann. Dadurch wird vermieden, dass in diesem Höhenbereich, in dem nur eine sehr geringe Menge an Knochenmaterial vorhanden ist, ein Teil dieses Knochenmaterials durch das Bohren entfernt wird. Das noch vorhandenen Knochenmaterial kann also praktisch vollständig erhalten werden. Damit erzielt man den Vorteil, dass Knochenmaterial an einer Stelle erhalten wird, an der es nicht oder nur sehr beschränkt neu gebildet werden könnte.

Ein Kerb- beziehungsweise Trenninstrument wird zu folgendem Zweck benutzt: Nach der Montage der Implantat-Anordnung im Kieferknochen wird ein Abstand des Zahnfleisches vom Kieferknochen benötigt, der durch das Weghalten des Zahnfleisches vom Kieferknochen geschaffen wurde. In diesen Abstand zwischen dem Kieferknochen und dem umgebenden Zahnfleisch kann nun das Kerb- beziehungsweise Trenninstrument eingebracht werden. Mittels des Kerb- beziehungsweise Trenninstrumentes erfolgt dann das Anbringen einer Kerbe oder Fuge am Kieferknochen mindestens in einer vorgesehenen Knochentrennebene. Die Knochentrennebene ist im Wesentlichen definiert durch die Lage der Berührungsebene, in welcher das proximale Implantat-Element das distale Implantat-Element berührt.

Zum Erzeugen des Abstandes um den Kieferknochen wird das Zahnfleisch, wie schon erwähnt, nicht operativ entfernt sondern lediglich chirurgisch geöffnet und vom Kieferknochen weggehalten. Das Kerb- beziehungsweise Trenninstrument wird bis zur Knochentrennebene eingebracht, wobei gegebenenfalls das Zahnfleisch angeschnitten werden kann. Dann wird der Kieferknochen in der Knochentrennebene mit Hilfe des Kerb- beziehungsweise Trenninstrumentes angeritzt beziehungsweise gekerbt. Die vollständige Trennung des distalen Kieferknochenbereiches vom proximalen Kieferknochenbereich erfolgt anschliessend im Laufe der Distraktion des distalen Kieferknochenbereiches, wie dies weiter oben beschrieben ist. Wurde der Kieferknochen nur angekerbt, so vertieft sich die Kerbe des Knochentrennbereichs hierbei zu einer Trennfuge, die sich anschliessend zu einem Spalt erweitert. In diesem Spalt bildet sich, wie ebenfalls weiter oben beschrieben, natürliches Knochenmaterial. Die temporären Bestandteile der Implantat-Anordnung können dann von den definitiven Bestandteilen der Implantat-Anordnung entfernt werden, und auf den definitiven Bestandteilen der Implantat-Anordnung wird anschliessend entweder ein Adapterteil mit einer Kronenaufnahme oder vorzugsweise die Krone selbst befestigt.

Ein bevorzugtes Spreizinstrument ist so ausgebildet, dass es Spreizflächen besitzt, die in kleinen Schritten oder stufenlos aus einer Ruhelage in eine Wirklage bringbar sind. In der Ruhelage bewirken die Spreizflächen keine Spreizung der Aushöhlung des Kieferknochens, während sie in der Wirkstellung die Aushöhlung des Kieferknochens aufspreizen.

In einer ersten Ausbildung weist das Spreizinstrument eine Spreizhülse mit einer Ausnehmung auf und wird über den distalen Ansatz des proximalen Implantat-Elementes geschoben. Die Spreizhülse ist aussen durch die Spreizfläche zulaufend, zum Beispiel konisch, begrenzt. Zum Spreizen wird die Spreizhülse einfach in proximaler Richtung über den Ansatz des proximalen Implantat-Elementes bis auf eine Schulter desselben geschoben. Die Spreizhülse ist an ihrem im Gebrauch distalen Ende an einem Griffteil befestigt.

Von der Spreizfläche können ausserdem zwei mindestens annähernd diametral angeordnete Schneiden nach aussen ragen. Die Schneiden können im Querschnitt gesehen unter einem Winkel angeordnet sein, so dass sie im Gebrauch etwa tangential in Richtung des Bogens des Kieferknochens verlaufen. Die Schneiden können einen bei Gebrauch distalen Bereich, in dem ihre Schneidkanten parallel zur Achse der Ausnehmung und damit bei Gebrauch parallel zur

Implantat-Längsachse verlaufen, aufweisen. An diesen distalen Bereich der Schneiden kann sich ein bei Gebrauch proximaler Bereich der Schneiden anschliessen, in dem die Schneidkanten in proximaler Richtung zulaufen; ein solcher zulaufender Bereich erleichtert die Einführung des Spreizinstrumentes. Mit Hilfe der Schneiden werden zwei saubere Schnitte angebracht, die das Spreizen erleichtern und einen Gewaltbruch verhindern, die aber anschliessend wieder verwachsen.

In einer zweiten Ausbildung des Spreizinstrumentes ist die Spreizfläche der Spreizhülse in Segmente aufgeteilt. Die Segmente befinden sich in der Ruhelage der Spreizfläche parallel und angrenzend an den Ansatz des proximalen Implantat-Elementes. In der Wirklage sind die Segmente mindestens teilweise beziehungsweise örtlich von diesem Ansatz beabstandet und spreizen so den Kieferknochen von diesem Ansatz weg.

Spreizinstrumente können gegebenenfalls auch benutzt werden, um schon vor dem Einschrauben des proximalen Implantat-Elementes den proximalen Kieferknochenbereich zu spreizen, nachdem dort eine kleindurchmessrige Bohrung erzeugt worden ist; dadurch verringert man die Menge Knochenmaterial, die durch Bohren zerstört wird und verloren geht.

Kerb- bzw. Trenninstrumente, die beim Implantieren von Implantat-Anordnungen für Zahnersatzteile benutzbar sind, können verschieden ausgebildet sein. Ein bevorzugtes Kerb- beziehungsweise Trenninstrument ist nach der Erfindung ist als Zange ausgebildet. Die Zange kann verschiedene, beispielsweise auswechselbare Einsätze besitzen, insbesondere einen Kerbeinsatz und einen Schneideinsatz.

Vorzugsweise besitzt das neue Kerb- beziehungsweise Trenninstrument eine Vorrichtung, welche es erlaubt, die Kerb- beziehungsweise Schneideinsätze in Richtung der Implantat-Längsachse beziehungsweise höhenmässig in der Knochentrennebene zu positionieren

Weitere Einzelheiten und Vorteile der Erfindung werden im Folgenden an Hand eines Ausführungsbeispieles und mit Bezug auf die Zeichnung ausführlich beschrieben. Es zeigen:
- **Fig. 1A**: ein erstes Beispiel einer Implantat-Anordnung nach der Erfindung, in einem die Implantat-Längsachse enthaltenden Schnitt;
- **Fig. 1B**: das proximale Implantat-Element der in Fig. 1A dargestellten Implantat-Anordnung;
- **Fig. 1C**: das distale Implantat-Element der in Fig. 1A dargestellten Implantat-Anordnung;
- **Fig. 1D**: das Hilfselement der in Fig. 1A dargestellten Implantat-Anordnung;
- **Fig. 2A bis 2G**: die Montage eines Zahnersatzteiles, das eine Implantat-Anordnung gemäss Fig. 1A enthält.
- **Fig. 3A**: ein zweites Beispiel einer Implantat-Anordnung nach der Erfindung, in einem die Implantat-Längsachse enthaltenden Schnitt;
- **Fig. 3B**: ein proximales Implantat-Element der in Fig. 3A dargestellten Implantat-Anordnung;
- **Fig. 3C**: ein distales Implantat-Element der in Fig. 3A dargestellten Implantat-Anordnung;
- **Fig. 3D**: ein erstes Hilfselement der in Fig. 3A dargestellten Implantat-Anordnung;
- **Fig. 3E**: ein zweites Hilfselement der in Fig. 3A dargestellten Implantat-Anordnung;
- **Fig. 3F**: ein Zwischenelement der in Fig. 3A dargestellten Implantat-Anordnung;
- **Fig. 4A bis 4G**: die Montage eines Zahnersatzteiles, das eine Implantat-Anordnung gemäss Fig. 3A enthält;
- **Fig. 5**: eine auf einer Implantat-Anordnung nach der Erfindung montierte Krone;
- **Fig. 6A**: ein erstes Spreizinstrument, in einer seitlichen Ansicht;
- **Fig. 6B**: das in Fig. 6A dargestellte Spreizinstrument, in einer Ansicht von unten;
- **Fig. 6C**: eine Spreizhülse des in den Fig. 6A und 6B dargestellten Spreizinstrumentes, in einer seitlichen Ansicht;
- **Fig. 6D**: eine Spreizhülse für ein zweites Spreizinstrument, mit Spreizflächen in ihrer Ruhelage, in stark vereinfachter Darstellung;
- **Fig. 6E**: das in Fig. 6D dargestellte Spreizinstrument, mit den Spreizflächen in ihrer Wirklage, in gleicher Darstellung wie Fig. 6E;
- **Fig. 7A**: ein Kerb- beziehungsweise Trenninstrument, in einer seitlichen Ansicht;
- **Fig. 7B**: ein erstes Einsatzpaar für das in Fig. 7A dargestellte Kerb- und Trenninstrument; und
- **Fig. 7C**: ein zweites Einsatzpaar für das in Fig. 7A dargestellte Kerb- und Trenninstrument.

Im nachfolgenden Teil der Beschreibung sind gleiche oder ähnliche Bestandteile Teile jeweils mit gleichen Bezugszeichen versehen, auch wenn sie in Einzelheiten verschieden ausgebildet sind. Die Darstellungen, insbesondere von Kieferknochen und Zahnfleisch, sind schematisiert und mindestens teilweise nicht massstäblich.

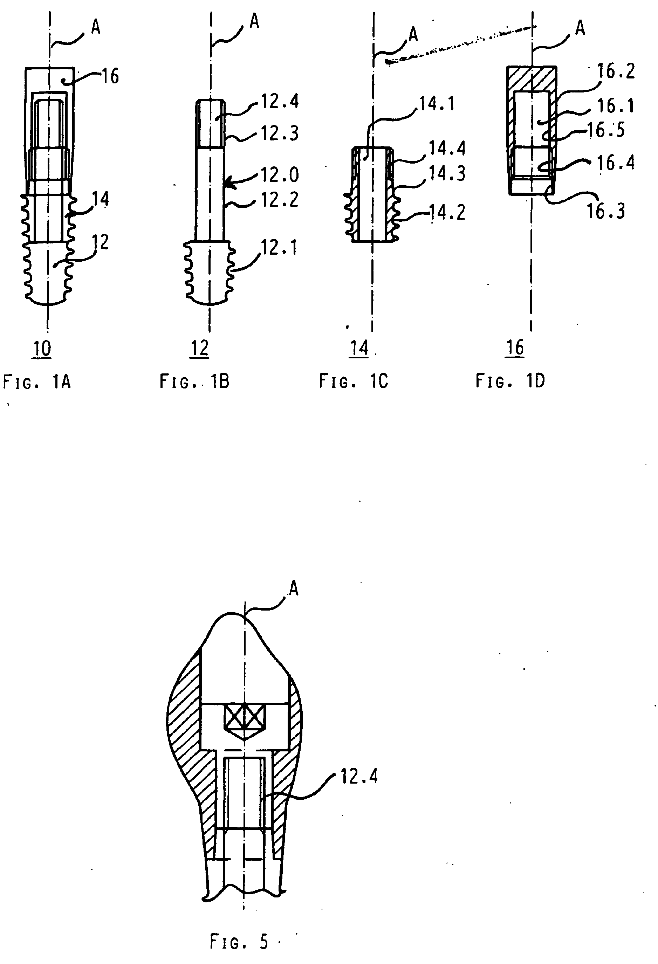

Die in **Fig. 1A** dargestellte Implantat-Anordnung **10** besitzt eine Implantat-Längsachse **A** und umfasst ein proximales Implantat-Element **12,** ein distales Implantat-Element **14** und ein Hilfselement **16.**

Das proximale Implantat-Element **12** gemäss **Fig. 1B** ist dazu bestimmt, in einen proximalen Kieferknochenbereich eingeschraubt zu werden. Das proximale Implantat-Element **12** besitzt einen sich in distaler Richtung erstreckenden Ansatz **12.0** und einen mit einem Aussengewinde beziehungsweise Implantatgewinde versehenen Gewindeabschnitt **12.1.** Als Implantatgewinde kommt beispielsweise das in der EP-A-1 112 722**-A2** beschriebene selbstschneidende, zur Befestigung von Implantaten in Knochenbereichen besonders geeignete, Aussengewinde in Frage. Der Ansatz **12.0** des proximalen Implantat-Elementes **12** weist, ausgehend vom Gewindeabschnitt **12.1**, einen ersten, zylindrischen Abschnitt **12.2** und daran anschliessend einen zweiten Abschnitt **12.3** auf. Der Abschnitt **12.3** bildet einen distalen Endbereich, der als Kronenträger **12.4** vorgesehen ist. Zu diesem Zwecke ist er mit einem Aussengewinde versehen.

Das in **Fig. 1C** dargestellte distale Implantat-Element **14** ist dazu bestimmt, angrenzend an das proximale Implantat-Element **12** in einen distalen Kieferknochenbereich eingeschraubt zu werden. Das distale Implantat-Element **14** besteht im Wesentlichen aus einer Gewindehülse mit einer zylindrischen Ausnehmung **14.1**, einem ersten, proximalen Gewindeabschnitt **14.2**, einem in distaler Richtung zulaufenden Abschnitt **14.3** ohne Aussengewinde und einem zweiten, distalen Gewindeabschnitt **14.4**. Der erste, proximale Gewindeabschnitt **14.2** besitzt ein Aussengewinde beziehungsweise Implantatgewinde, beispielsweise ein Implantatgewinde, wie es in der EP-A- 1 112 722**-A2** beschrieben ist, welches in den distalen Kieferknochenbereich einschraubbar ist. Der Zweck des zulaufenden Abschnittes **12.3** wird weiter unten erklärt. Der zweite, distale Gewindeabschnitt 14.4 besitzt ein Aussengewinde, dessen Aussendurchmesser geringer ist als der Aussendurchmesser des Implantatgewindes des ersten, proximalen Gewindeabschnittes **14.2.**

Das in **Fig. 1D** genauer dargestellte Hilfselement **16,** das als Zug- und Stellglied für die Distraktion des distalen Kieferknochenbereiches benutzt wird, ist durch eine Zughülse gebildet, für die ebenfalls das Bezugszeichen **16** verwendet wird. Die Zughülse **16** weist eine von ihrem proximalen Ende ausgehende, am distalen Ende verschlossene Ausnehmung **16.1** auf und ist aussen durch eine Zylindermantelfläche **16.2** begrenzt; dies ist aber nicht zwingend, die äussere Begrenzungsfläche der Zughülse **16** könnte auch anders geformt, beispielsweise leicht zulaufend, sein. Die Ausnehmung **16.1** der Zughülse **16** weist einen proximalen, in distaler Richtung zulaufenden Abschnitt **16.3,** einen proximalen Gewindeabschnitt **16.4 und** einen zylindrischen Abschnitt **16.5** auf. Der zulaufende Abschnitt **16.3** kommt im montierten Zustand der Implantat-Anordnung **10** gemäss **Fig. 1A** am zulaufenden Abschnitt **14.3** des distalen Implantat-Elementes **14** zur Anlage. Der proximale Gewindeabschnitt **16.4** ist im montierten Zustand der Implantat-Anordnung **10** gemäss **Fig. 1A** mit dem komplementären Gewindeabschnitt **14.4** des distalen Implantat-Elementes **14** verschraubt. Der zylindrische Abschnitt **16.5** umgibt den Abschnitt **12.3** des Ansatzes **12.0** des proximalen Implantat-Elementes **12**.

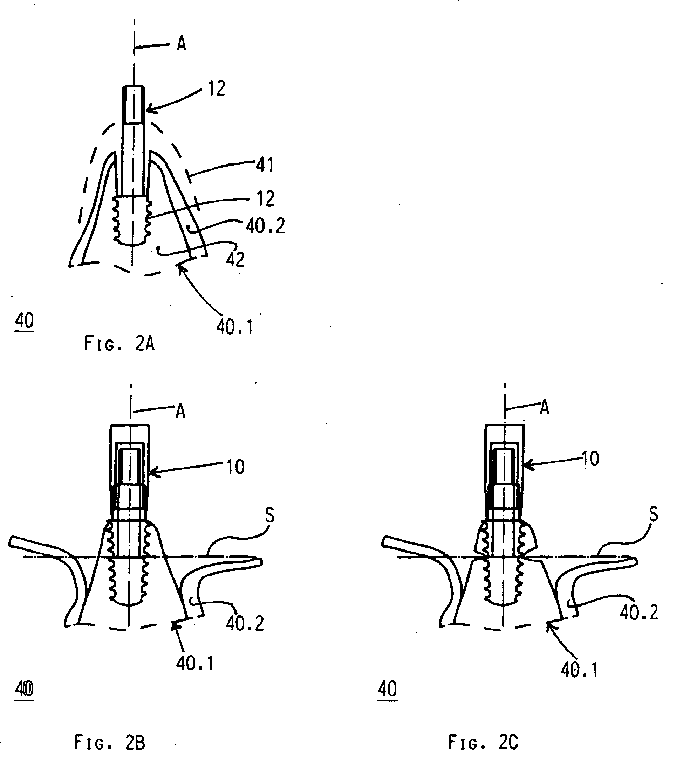

Die **Fig. 2A** bis **2G** veranschaulichen die Vorgänge bei der Montage eines Zahnersatzes mit einer Implantat-Anordnung **10,** wie sie in **Fig. 1A** dargestellt ist.

**Fig. 2A** zeigt einen Kieferbereich **40** eines Patienten, mit einem Kieferknochen **40.1** und mit den Kieferknochen **40.1** umgebendem Zahnfleisch **40.2**. Der Kieferknochen **40.1** ist von Knochenschwund betroffen; die ungefähre Grösse des ursprünglichen Kieferknochens ist durch eine gestrichelte Linie **41** angedeutet. Das proximale Implantat-Element **12** ist bereits implantiert, das heisst in einem proximalen Kieferknochenbereich **42** eingeschraubt.

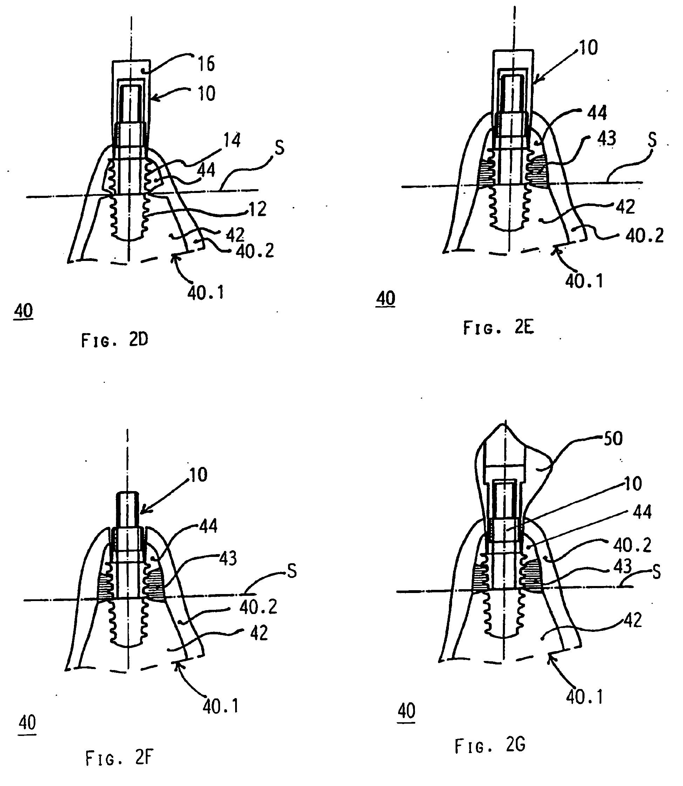

**Fig. 2B** zeigt den Kieferbereich **40** mit dem im proximalen Kieferknochenbereich **42** implantierten proximalen Implantat-Element **12** und dem im distalen Kieferknochenbereich **44** implantierten distalen Implantat-Element **14**. Das distale Implantat-Element **14** wird hierbei so tief in den distalen Kieferknochenbereich **44** eingeschraubt, dass seine Vorderfront an einer Schulter des proximalen Implantat-Elementes zur Anlage kommt. Die Schulter definiert diejenige Stelle beziehungsweise eine in **Fig. 2C** und **2D** eingezeichnete Knochentrennebene **S**, an welcher die Trennung des distalen Kieferknochenbereiches **44** vom proximalen Kieferknochenbereich **42** stattfinden wird. Das Implantieren der Implantat-Elemente **12, 14** ist der einzige Vorgang bei der Montage des Zahnersatzteiles **1**, bei welchem eine Operationswunde entsteht. Als nächstes wird, immer noch gemäss **Fig. 2B,** das Hilfselement beziehungsweise die Zughülse **16** mit ihrem proximalen Gewindeabschnitt **16.4** auf den distalen Gewindeabschnitt **14.4** des distalen Implantat-Elementes **14** aufgeschraubt, wonach die zulaufenden Fläche der Zughülse **16** satt beziehungsweise dichtend an der zulaufenden Fläche des Implantat-Elementes **14** anliegt.

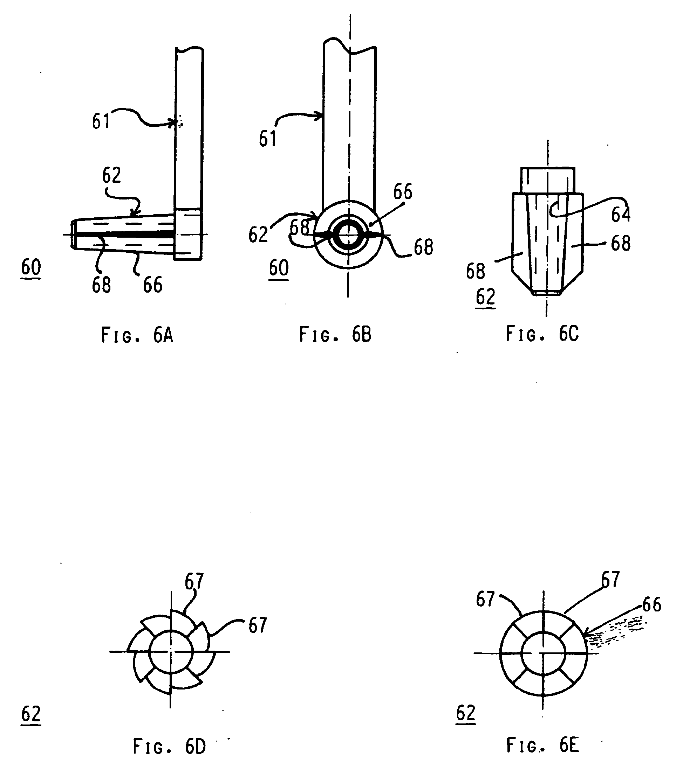

Vor der Montage des distalen Implantat-Elementes **14** wurde das Zahnfleisch **40.2** chirurgisch geöffnet und vom Kieferknochen **40.1** weggehalten, wie es in **Fig. 2C** dargestellt ist. Sodann wurde der distale Kieferknochenbereich **44** mit Hilfe eines Spreizinstrumentes **60** aufgespreizt und das distale Implantat-Element **14** montiert. Das Spreizinstrument **60** ist weiter unten mit Bezug auf **Fig. 6** beschrieben.

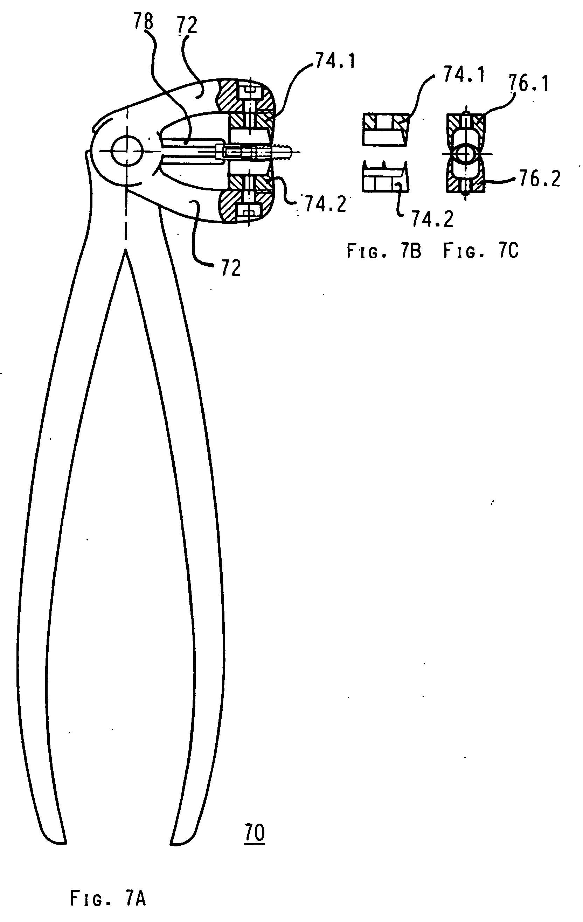

**Fig. 2C** zeigt, wie anschliessend mit Hilfe eines Kerb- beziehungsweise Trenninstrumentes **70** der Kieferknochen **40.1** in der Knochentrennebene **S** angekerbt beziehungsweise mindestens teilweise getrennt worden ist. Das Kerb- und Trenninstrument **70** ist weiter unten mit Bezug auf **Fig. 7** beschrieben.

**Fig. 2D** zeigt den Zustand nach Erzeugung einer Kerbe und nach Entfernung des Kerb- beziehungsweise Trenhinstrumentes **70**.

Nun wird die auf das distale Implantat-Element **14** aufgeschraubte Zughülse **16** weiter gedreht; dadurch würde die Schraubverbindung zwischen distalem Implantat-Element **14** und Zughülse **16** weiter angezogen, falls dies noch möglich wäre. Da aber die Zughülse **16** bereits so weit wie möglich mit dem distalen Implantat-Element **14** verschraubt ist und sich das distale Implantat-Element **14** selbst nicht weiter in den Kieferknochen **40.1** einschrauben lässt, erfolgt beim weiteren Drehen der Zughülse **16** eine Distraktion des distalen Kieferknochenbereiches **44** weg vom proximalen Kieferknochenbereich **42**. Das distale Implantat-Element **12** wird hierbei nicht mit-distrahiert.

Die zu Beginn der Distraktion nur ansatzweise bestehende Trennfuge beziehungsweise als Kerbe zwischen dem proximalen Kieferknochenbereich **42** und dem distalen Kieferknochenbereich **44** vervollständigt sich und erweitert sich schrittweise zu einem immer breiteren Spalt **43**. **Fig. 2E** zeigt diesen Vorgang in einem Zustand, in welchem der Spalt **43** etwa seine definitive Breite erreicht hat. Im Spalt **43** bildet sich daraufhin in einem Zeitintervall von Tagen, Wochen, ggfs. sogar Monaten nach und nach natürliches Knochenmaterial; auf diese Weise erfolgt ein Knochenaufbau beziehungsweise ein Ersatz des durch Knochenschwund verlorenen Knochenmaterials.

Das Drehen der Zughülse **16**, das die Distraktion verursacht, wird in Schritten von etwa 10° bis 40° in zeitlichen Abständen von einigen Stunden bis zu einigen Tagen oder Wochen durchgeführt; dies hängt von der Reaktion des einzelnen Patienten auf die bisher beschriebenen Vorgänge ab. Dieses Drehen der Zughülse **16** kann durch eine Fachperson, das heisst einen Zahnarzt oder eine Dentalhygienikerin, oder durch den Patienten selbst oder eine nicht-professionelle Hilfskraft erfolgen.

Bei dem in **Fig. 2E** dargestellten Endzustand der oben beschriebenen Distraktion des distalen Kieferknochenbereiches **44** wurde der distale Kieferknochenbereich **44** so weit in distaler Richtung verschoben, dass - zusammen mit dem im Spalt **43** neu gebildeten Knochenmaterial - ein Kieferknochen entstanden ist, der etwa dem ursprünglichen Kieferknochen gemäss der gestrichelten Linie **41** in **Fig. 2A** entspricht. Die endgültige Weite des Spaltes **43** beziehungsweise die Dicke des neuen Knochenmaterials kann etwa im Bereich von etwa 0.5 mm bis etwa 5 mm liegen.

Als Nächstes werden die provisorischen Bestandteile der Implantat-Anordnung **10** von den definitiven Bestandteilen der Implantat-Anordnung **10** entfernt. Definitiv implantiert bleiben beim vorliegenden Ausführungsbeispiel das proximale Implantat-Element **12** und das distale Implantat-Element **14**. **Fig. 2F** zeigt den Zustand, bei welchem das einzige Hilfselement **16**, das heisst die Zughülse, entfernt worden ist.

Schliesslich wird gemäss **Fig. 2G** eine Krone **50** auf dem als Kronenaufnahme ausgebildeten distalen Endbereich des proximalen Implantat-Elementes **12** befestigt. Die Befestigung erfolgt hier durch Aufschrauben der Krone **50**, es können hierzu aber auch andere Befestigungsmittel als eine Gewindeverbindung vorgesehen sein. Im vorliegenden Ausführungsbeispiel spannen das proximale Implantat-Element **12** und die Krone **50** das distale Implantat-Element **14** ein, wodurch die Stabilität des Zahnersatzteiles **1** in sich gewährleistet ist. Dies wird erreicht, indem die Krone **50** eine Zugkraft auf das proximale Implantat-Element **12** und eine Druckkraft auf das distale Implantat-Element **14** ausübt.

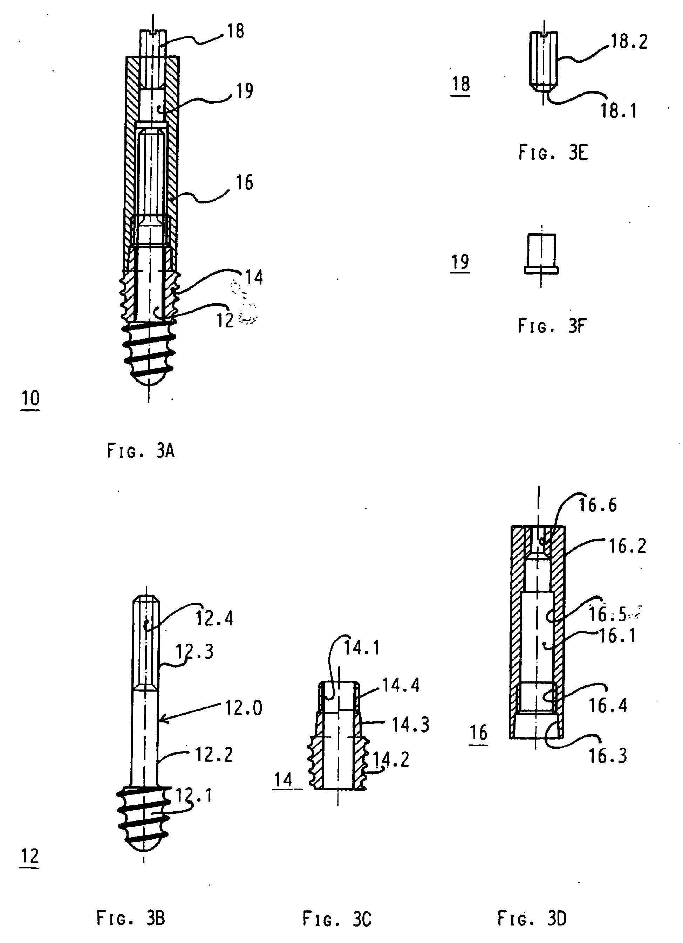

Das in **Fig. 3A** dargestellte zweite Ausführungsbeispiel der Implantat-Anordnung **10** nach der Erfindung besitzt eine Implantat-Längsachse **A** und umfasst ein proximales Implantat-Element **12**, ein distales Implantat-Element **14**, ein Hilfselement **16**, ein weiteres Hilfselement **18** und ein Zwischenelement **19**.

Das proximale Implantat-Element **12** gemäss **Fig. 3B** ist gleich ausgebildet und im montierten Zustand gleich angeordnet wie das in **Fig. 1B** dargestellte proximale Implantat-Element.

Das in **Fig. 3C** dargestellte distale Implantat-Element **14** ist gleich ausgebildet und im montierten Zustand gleich angeordnet wie das in **Fig. 1C** dargestellte distale Implantat-Element.

Das in **Fig. 3D** genauer dargestellte Hilfselement **16**, das als Zugelement bei der Verschiebung des distalen Implantat-Elementes **14** benutzt wird, ist ähnlich ausgebildet wie das in **Fig. 1D** dargestellte Hilfselement **16**. Es ist durch eine Zughülse gebildet, für die ebenfalls das Bezugszeichen **16** verwendet wird. Die Zughülse **16** weist hier eine durchgehende Ausnehmung **16.1** auf. Wie die Zughülse gemäss **Fig. 1D** ist auch die Zughülse **16** gemäss **Fig. 3D** aussen durch eine Zylindermantelfläche **16.2** begrenzt; dies ist aber nicht zwingend, die distale Begrenzungsfläche der Zughülse **16** könnte auch leicht konisch sein. Die Ausnehmung **16.1** der Zughülse **16** weist einen ersten, in distaler Richtung zulaufenden Abschnitt **16.3**, einen proximalen Gewindeabschnitt **16.4**, einen zylindrischen Abschnitt **16.5** und einen distalen Gewindeabschnitt **16.6** auf. Der zulaufende Abschnitt **16.3** kommt im montierten Zustand der Implantat-Anordnung **10** gemäss **Fig. 3A** am zulaufenden Abschnitt **14.3** des distalen Implantat-Elementes **14** zur Anlage. Der proximale Gewindeabschnitt **16.4** ist im montierten Zustand der Implantat-Anordnung **10** gemäss **Fig. 3A** mit dem komplementären Gewindeabschnitt **14.4** des distalen Implantat-Elementes **14** verschraubt. Der zylindrische Abschnitt **16.5** umgibt den Abschnitt **12.3** des Ansatzes des proximalen Implantat-Elementes **12.** Der distale Gewindeabschnitt **16.6** dient im montierten Zustand der Implantat-Anordnung **10** gemäss **Fig. 1** zur Aufnahme des zweiten Hilfselementes **18.**

Das in **Fig. 3E** dargestellte weitere Hilfselement **18,** das als Stellglied für die Distraktion des distalen Kieferknochenbereiches **44** benutzt wird, ist als ebenfalls mit **18** bezeichnete Stellschraube ausgebildet. Die Stellschraube **18** weist eine ihre Endfläche bildende Wirkfläche **18.1** und ein Aussengewinde **18.2** auf, das komplementär zum Innengewinde des distalen Gewindeabschnittes **16.6** der Zughülse **16** ist.

Das in **Fig. 3F** dargestellte Zwischenelement **19** ist im montierten Zustand der Implantat-Anordnung **10** gemäss **Fig. 3A** im Proximalen der Zughülse **16** zwischen der Wirkfläche **18.1** des Stellgliedes **18** und der Druckfläche des proximalen Implantat-Elementes **12** angeordnet und dient als Dichtungselement.

Die **Fig. 4A** bis **4H** veranschaulichen die Vorgänge bei der Montage eines Zahnersatzteils **1** mit einer Implantat-Anordnung **10**, wie sie in **Fig. 3A** dargestellt ist.

**Fig. 4A** zeigt den Zustand nach dem Implantieren des proximalen Implantat-Elementes **12** und **Fig. 4D** den Zustand nach dem Implantieren des distalen Implantat-Elementes **14,** nach der Befestigung des Hilfselementes **16** auf dem distalen Implantat-Element **14** und nach dem vorläufigen Festschrauben des weiteren Hilfselementes **18**.

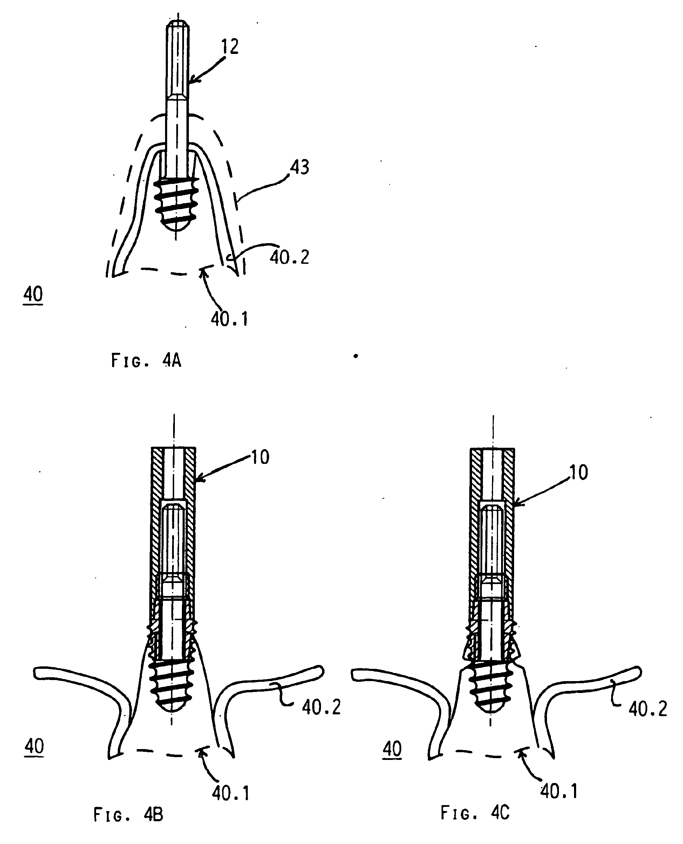

**Fig. 4B** zeigt den Zustand mit dem vom Kieferknochen **40.1** weggehaltenen Zahnfleisch **40.2** vor dem Anbringen der Kerbe in der Knochentrennebene **S** des Kieferknochens **40.1. Fig. 4C** zeigt den Zustand mit dem weggehaltenen Zahnfleisch **40.2** nach dem Anbringen der Kerbe im Kieferknochens **40.1**. **Fig. 4D** zeigt den Zustand mit dem Zahnfleisches **40.2** annähernd in seiner ursprünglichen Konfiguration.

Nun wird die Stellschraube **18** weiter in die Zughülse **16** eingeschraubt. Beim Anziehen der Stellschraube **18**, das in Schritten von etwa 10° bis 40° in zeitlichen Abständen von einigen Stunden bis zu einigen Tagen erfolgt, beaufschlagt die Wirkfläche der Stellschraube **16** über das Zwischenelement **19** die Druckfläche des proximalen Implantat-Elementes **12**. Da das proximale Implantat-Element **12** trotz dieses Druckes nicht weiter in proximaler Richtung verschiebbar ist, bewirkt das Anziehen der Stellschraube **18**, dass sich die Zughülse **16** in distaler Richtung verschiebt und hierbei das distale Implantat-Element **14** und den distalen Kieferknochenbereich **44** mitnimmt. Bei der Verschiebung des distalen Implantat-Elementes **14** ist dieses am ortsfest im proximalen Kieferbereich **42** verbleibenden proximalen Implantat-Element **12** geführt. Die bisher nur ansatzweise bestehende Trennfuge beziehungsweise Kerbe zwischen dem proximalen Kieferknochenbereich **42** und dem distalen Kieferknochenbereich **44** vervollständigt sich und erweitert sich dann schrittweise zum immer breiteren Spalt **43. Fig. 4E** zeigt diesen Vorgang in einem Zustand, in welchem der Spalt **43** etwa seine halbe definitive Breite erreicht hat. Im Spalt **43** bildet sich nach und nach natürliches Knochenmaterial; auf diese Weise erfolgt ein Knochenaufbau beziehungsweise ein Ersatz des durch Knochenschwund verlorenen Knochenmaterials.

Ein Vorteil dieses zweiten Ausführungsbeispiels der Implantat-Anordnung **10** mit der Zughülse **16** und der Stellschraube **18** kann darin gesehen werden, dass Markierungen an Zughülse **16** und Stellschraube **18** angebracht werden können, die es erlauben, das Mass der jeweiligen relativen Drehung leichter festzustellen, was insbesondere dann erwünscht ist, wenn dieses Drehen dem Patienten überlassen wird.

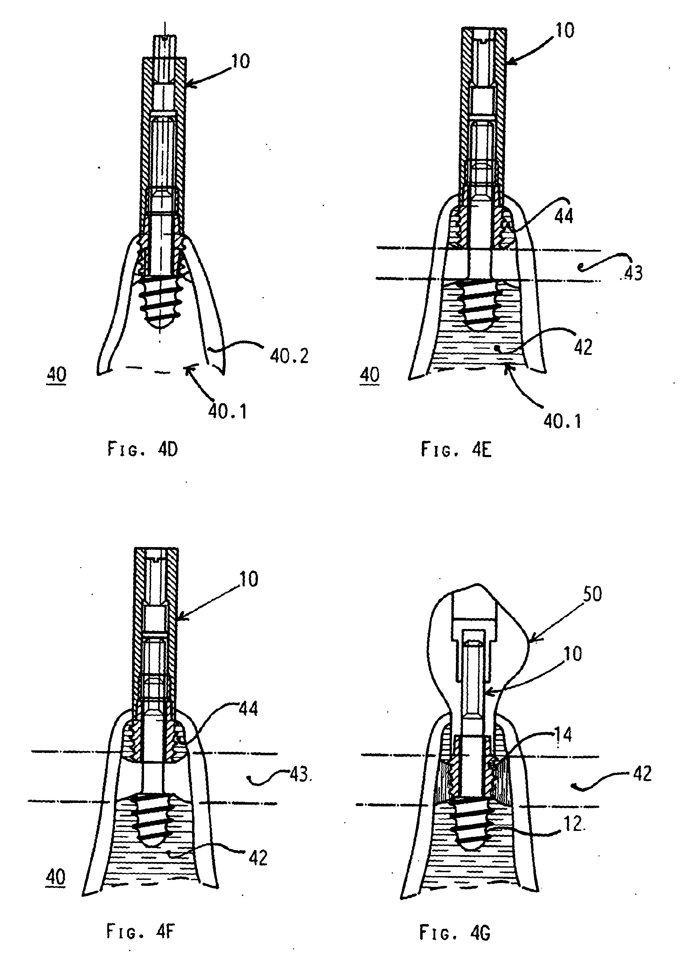

In **Fig. 4F** ist der Endzustand der oben beschriebenen Distraktion des distalen Kieferknochenbereiches **44** dargestellt; dieser wurde durch das in ihm verankerte distale Implantat-Element **14** so weit in distaler Richtung verschoben, dass - zusammen mit dem im Spalt **43** neu gebildeten Knochenmaterial - ein Kieferknochen entstanden ist, der etwa dem ursprünglichen Kieferknochen gemäss der gestrichelten Linie **41** in **Fig. 4A** entspricht. Die endgültige Weite des Spaltes **43** beziehungsweise die Dicke des neuen Knochenmaterials kann etwa im Bereich von etwa 0.5 mm bis etwa 5 mm liegen.

**Fig. 4G** zeigt schliesslich das nach der Distraktion des distalen Kieferknochenbereiches **44** wieder bis zur Schulter des proximalen Implantat-Elementes **12** eingeschraubte distale Implantat-Element **14**.

**Fig.4G** zeigt einen Zustand, bei welchem die provisorischen Bestandteile der Implantat-Anordnung **10** von den definitiven Bestandteilen der Implantat-Anordnung **10** entfernt wurden. Definitiv implantiert bleiben das proximale Implantat-Element **12** und das distale Implantat-Element **14**. Entfernt werden das Hilfselement, das heisst die Zughülse **16** einschliesslich des darin angeordneten Zwischenelementes **19** und das weitere Hilfselement, nämlich die Stellschraube **18.** Das Lösen temporärer Bestandteile der Implantat-Anordnung **10** kann generell schonend mit Hilfe von Ultraschall erfolgen.

Schliesslich wird, ebenfalls gemäss **Fig. 4G**, die Krone **50** auf dem als Kronenaufnahme ausgebildeten distalen Endbereich beziehungsweise Kronenträger **12.4** des proximalen Implantat-Elementes **12** befestigt, in gleicher Weise, wie dies weiter oben mit Bezug auf **Fig. 2G** beschrieben ist.

**Fig. 5** zeigt ausschnittweise ein Zahnersatzteil **1** mit einer Krone **50,** die auf dem Kronenträger **12.4** des proximalen Implantat-Elementes **12** befestigt ist.

Die **Fig. 6A** bis **6C** zeigen ein erstes Ausführungsbeispiel des Spreizinstrumentes 60. Das Spreizinstrument **60** wird insbesondere verwendet, um eine kteindurchmessrige Bohrung oder Aushöhlung den Kieferknochens **40.1** aufzuspreizen, damit ein den Ansatz des proximalen Implantat-Elements **12** umgebender Abstand entsteht, so dass das distale Implantat-Element **14** eingeschraubt werden kann. Zuvor wird das Zahnfleisch **40.2** chirurgisch geöffnet und vom Kieferknochen **40.1** ferngehalten. Das Spreizinstrument **60** besteht im Wesentlichen aus einem Handgriff **61** und einer an diesem Handgriff **61** befestigten Spreizhülse **62**. Die Spreizhülse **62** weist eine in Richtung der Hülsenlängsachse verlaufende Ausnehmung **64** auf, die im Querschnitt komplementär zu einem Hülsenführungsteil des Implantat-Elementes **12** ausgebildet ist. Das Hülsenführungsteil ist im vorliegenden Ausführungsbeispiel durch den Ansatz des Implantat-Elementes **12** gebildet, welches sich in der kleindurchmessrigen Bohrung oder Aushöhlung des distalen Kieferknochenbereiches **44** befindet. Aussen ist die Spreizhülse **62** durch eine Spreizfläche **66** begrenzt, welche vorzugsweise in proximaler Richtung zulaufend ausgebildet ist. Zum Aufspreizen des Kieferkno chens **40.1** beziehungsweise des distalen Kieferknochenbereiches **44**, ausgehend von Ansatz des proximalen Implantat-Elementes **12**, wird das Spreizinstrument **60** so gehandhabt, dass die Spreizfläche **66** aus einer Ruhelage in eine Wirklage gebracht wird, entweder durch leichte Rotation oder durch Verschiebung in Richtung der Implantat-Längsachse. In der Ruhelage der Spreizfläche **66** liegt die Spreizhülse **60** mit ihrer Ausnehmung am Ansatz beziehungsweise Hülsenführungsteil des proximalen Implantat-Elementes **12** an, befindet sich jedoch noch in der schon bestehenden Aushöhlung im Kieferknochen **40.1**. Dann wird die Spreizfläche **66** nach und nach in ihre Wirklage gebracht, indem die Spreizhülse **62** in proximaler Richtung vom Ansatz beziehungsweise Hülsenführungsteil weggespreizt wird und hierbei im Wesentlichen den distalen Kieferknochenbereich **44** spreizt. Die Verschiebung der Spreizhülse **62** endet, wenn ihre Vorderfläche **63** an der Schulter des Implantat-Elementes **12**, die an das Implantatgewinde des Implantat-Elementes **12** grenzt, zum Anschlag kommt.

Vorzugsweise weist die Spreizfläche **66** der Spreizhülse **62** radial nach Aussen ragende Schneiden **68** auf. Solche Schneiden **68** erstrecken sich mindestens über einen Teil der Höhe der Spreizhülse **62** und sind vorzugsweise mindestens an ihrem proximalen Endbereich **69** in proximaler Richtung zulaufend ausgebildet. Im Allgemeinen sind zwei solcher Schneiden **68** angeordnet, die mindestens annähernd diametral angeordnet sind. Spreizhülsen **62** mit Schneiden **68** werden vorzugsweise nur geradlinig in ihre Wirklage gebracht.

**Fig. 6D** und **6E** zeigen ein zweites Ausführungsbeispiel des Spreizinstrumentes **60.** Die Spreizfläche **66** ist hierbei durch Spreizsegmente **67** gebildet, die beispielsweise in einer Ruhelage in Richtung der Implantat-Längsachse A verlaufen und dann nach Aussen in eine Wirklage verstellt werden, in welcher der Kieferknochen **40.1** gespreizt ist. Das Verschieben der Spreizsegmente **67** kann mechanisch oder fluidbetätigt erfolgen.

**Fig. 7A** zeigt das Kerb- beziehungsweise Trenninstrument **70**. Es handelt sich hierbei um eine Zange mit zwei Backen **72**. Es könnte aber auch eine Zange mit mehreren circumferential verteilten Backen benutzt werden.

Diese Backen **72** können mit verschiedenen Einsatzpaaren bestückt werden können. Ein erstes Einsatzpaar **74.1, 74.2,** das in **Fig. 7A** und **Fig. 7B** dargestellt ist, dient zum Ritzen beziehungsweise Kerben des Kieferknochens in Richtung der Knochentrennebene **S**. Ein zweites Einsatzpaar **76.1 76.2,** das in **Fig. 7C** dargestellt ist, dient zum Trennen beziehungsweise Schneiden des Kieferknochens **40.1** seitlich und in Richtung der Knochentrennebene **S**. Eine Vorrichtung **78** in der Art einer Distanzhülse, dargestellt in **Fig. 7A,** dient dazu, die Einsätze genau zu positionieren, so dass das Kerben beziehungsweise Trennen an den vorgesehenen Stellen stattfindet.

Als Materialien für die Implantat-Elemente **12** und **14** werden Werkstoffe eingesetzt, die dauerhaft biokompatibel und keinesfalls biodegradabel sind, beispielsweise Titan oder Titanlegierungen. Die Werkstoffe für das Hilfselement **16** und gegebenenfalls das weitere Hilfselement **18** müssen zwar eine genügende mechanische Festigkeit haben und in Gewindeverbindungen mit den Werkstoffen der Implantat-Elemente kombinierbar sein, hingegen sind die Ansprüche betreffend langfristiger Biokompatibilität geringer; im Allgemeinen werden Titan oder Chromlegierungen beziehungsweise Chromstähle benutzt. Das Zwischenelement **19**, das im vorliegenden zweiten Ausführungsbeispiel verwendet wird, besteht aus einem geeigneten und für solche Zwecke erprobten Kunststoff, beispielsweise aus Polyethylen.

Es wird ausdrücklich darauf hingewiesen, dass die oben beschriebenen Implantat-Anordnungen **10** lediglich als Beispiele zu betrachten sind. Im Rahmen der Patentansprüche sind zahlreiche andere Ausbildungen der erfindungsgemässen Implantat-Anordnung **10** möglich. So ist es insbesondere möglich, Implantat-Anordnungen zu schaffen, bei denen die Lagen von Innen- und Aussengewinde und/oder innen- und aussenliegenden beziehungsweise hülsenartigen und kernartigen Elementen gegenüber dem obigen Ausführungsbeispiel vertauscht sind; ferner kann der Kronenträger am distalen Implantat-Element befestigt werden oder es kann ein herkömmliches zusätzliches Adapterteil zur Befestigung der Krone verwendet werden.

## Patentansprüche

1. Implantat-Anordnung (**10**) für ein Zahnersatzteil (**1**), die dazu bestimmt ist, in Richtung einer Implantat-Längsachse (**A**) in einen Kieferknochen (**40.1**) implantiert zu werden, wobei die Implantat-Anordnung (**10**)
- ein proximales Implantat-Element **(12)** mit einem sich in distaler Richtung erstreckenden Ansatz **(12.0)** und einem äusseren Implantatgewindeabschnitt **(12.1),** das dazu bestimmt ist, in einen proximalen Kieferknochenbereich **(42)** des Kieferknochens **(40.1)** implantiert zu werden, und
- ein distales Implantat-Element (14) mit einer zylindrischen Ausnehmung (**14.1**), einem ersten, proximalen, äusseren Implantatgewindeabschnitt (**14.2**) und einem zweiten, distalen Gewindeabschnitt (**14.4**), das dazu bestimmt ist, in einen distalen Kieferknochenbereich (**44**) des Kieferknochens (**40.1**) durch eine Einschraubbewegung implantiert zu werden, wobei das distale Implantat-Element (**14**) koaxial auf dem erstreckenden Ansatz (**12.0**) des proximalen Implantat-Elementes (**12**) verdrehbar und verschiebbar ist,
aufweist, welche Implantat-Elemente (**12, 14**)zu einer Distraktion des distalen Kieferknochenbereiches **(44)** relativ zum proximalen Kieferknochenbereich **(42)** in Richtung der Implantat-Längsachse **(A)** ausgebildet sind,
**dadurch gekennzeichnet,**
**dass** der zweite, distale Gewindeabschnitt (**14.4**) des distalen Implantat-Elementes (**14**) ein Aussengewinde besitzt, dessen Aussendurchmesser geringer ist als der Aussendurchmesser des erste, proximalen, äusseren Gewindeabschnitts **(14.2),** so dass das distale Implantat-Element **(14)** so tief wie möglich in den distalen Kieferknochenbereich (**44**) bis zum Anschlag am proximalen Implantat-Element **(12)** einschraubbar ist.

2. Implantat-Anordnung (**10**) nach Anspruch **1**,
**dadurch gekennzeichnet,**
**dass** das in den distalen Kieferknochenbereich **(44)** einschraubbare distale Implantat-Element **(14)** nach dem Anschlag am proximalen Implantat-Element **(12)** weiter um die Implantat-Längsachse **(A)** rotierbar und hier-bei der distale Kieferknochenbereich. **(44)** relativ zum proximalen Kieferknochenbereich **(42)** in distaler Richtung verschiebbar ist, um die Distraktion durchzuführen und den distalen Kieferknochenbereich **(44)** um einen Spalt (43) vom proximalen Kieferknochenbereich **(42)** zu beabstanden.

3. Implantat-Anordnung **(10)** nach [Anspruch 1] einem der Ansprüche 1 bis **2**,
**dadurch gekennzeichnet,**
**dass** das proximale Implantat-Element (**12**) und das distale Implantat-Element (**14**) definitive Bestandteile der Implantat-Anordnung (**10**) und dazu bestimmt sind, nach dem Aufbau des Zahnersatzteiles (**1**) im Kieferknochen **(40.1)** befestigt zu verbleiben.

4. Implantat-Anordnung **(10)** nach einem der Ansprüche **1** bis **3**
**dadurch gekennzeichnet,**
**dass** sie Mittel (**16, 18**) aufweist, um, vorzugsweise nach einer mindestens teilweisen Trennung des distalen Kieferknochenbereiches (**44**) vom proximalen Kieferknochenbereich **(42),** das distale Implantat-Element (**14**) zu rotieren, um den distalen Kieferknochenbereich **(44)** relativ zum proximalen Kieferknochenbereich **(42)** zu distrahieren,

5. Implantat-Anordnung **(10)** nach Anspruch **4**,
**dass** mindestens ein Teil der zum Distrahieren des distalen KieferknochenBereiches (**44**) bestimmten Mittel (**16, 18**) temporäre Bestandteile der Implantat-Anordnung (**10**) sind, welche dazu bestimmt sind, nach dem Distrahieren des distalen Kieferknochenbereiches (**44**) von den definitiven Bestandteilen (**12, 14**) der Implantat-Anordnung (**10**) entfernt zu werden.

6. Implantat-Anordnung **(10)** nach einem der Ansprüche [3 bis 4] **4** bis **5**,
**dadurch gekennzeichnet,**
**dass** die zum Verschieben des distalen Kieferknochenbereiches (**44**) bestimmten Mittel ein Hilfselement (**16**) aufweisen, das dazu bestimmt ist, während des Distrahierens des distalen Kieferknochenbereiches (**44**) bewegungsmässig mit dem distalen Implantat-Element (**14**) solidarisch zu sein.

7. Implantat-Anordnung **(10)** nach Anspruch **6**,
**dadurch gekennzeichnet,**
**dass** die zum Verschieben des distalen Kieferknochenbereiches (**44**) bestimmten Mittel ein weiteres Hilfselement (**18**) aufweisen, das relativ zum Hilfselement (**16**) in Richtung der Implantat-Längsachse (**A**) bewegbar ist, wobei das weitere Hilfselement (**16**) dazu bestimmt ist, während des Distrahierens des distalen Kieferknochenbereiches (**44**) bewegungsmässig mit dem proximalen Implantat-Element **(12)** solidarisch zu sein.

8. Implantat-Anordnung **(10)** nach Anspruch **6** oder **7**,
**dadurch gekennzeichnet,**
**dass** das Hilfselement (**16**) als Zugglied ausgebildet und zwecks Distraktion des distalen Kiefernochenbereiches (**44)** verstellbar ist.

9. Implantat-Anordnung **(10)** nach Anspruch 7 oder 8,
**dadurch gekennzeichnet,**
**dass** das weitere Hilfselement (**18**) als Stellglied ausgebildet ist, das zwecks Distraktion des distalen Kieferknochenbereiches (**44**) verstellbar ist.

10. Implantat-Anordnung nach einem der Ansprüche **6** bis **9**,
**dadurch gekennzeichnet,**
**dass** das Hilfselement (**16**) und ggfs. das weitere Hilfselement (**18**) Markierungen aufweisen, um das Mass ihrer Verstellung anzuzeigen.

11. Implantat-Anordnung **(10)** nach Anspruch **4**,
**dadurch gekennzeichnet,**
**dass** das proximale Implantat-Element (**12**), das distale Implantat-Element (**14**) und die Mittel (**16, 18, 19**) zum Distrahieren des distalen Implantat-Elementes (**14**) so ausgebildet sind, dass die Implantat-Anordnung (**10**) dichtend im Kieferknochen (**40.1**) und in einem an den Kieferknochen (**40.1**) grenzenden Bereich von Zahnfleisch (**40.2**) montierbar ist.

12. Implantat-Anordnung **(10)** nach Anspruch **7**,
**dadurch gekennzeichnet,**
**dass** die Mittel (**16, 18**) zum Distrahieren des distalen Kieferknochenbereiches (**44**) ein als Dichtungselement wirkendes Hilfselement (**19**) umfassen, das vorzugsweise zwischen dem proximalen Implantat-Element (**12**) und dem weiteren Hilfselement (**18**) angeordnet ist.

13. Implantat-Anordnung (**10**) nach Anspruch **6**,
**dadurch gekennzeichnet,**
**dass** das Hilfselement (**16**) eine, vorzugsweise zulaufende, Dichtungsfläche besitzt, mit welcher es im montierten Zustand an einer komplementären Dichtungsfläche der Implantat-Anordnung (10) dichtend anliegt.

14. Implantat-Anordnung (**10**) nach Anspruch **1**,
**dadurch gekennzeichnet,**
**dass** sie eine Führungsanordnung besitzt, mit einer ersten Führungsfläche am proximalen Implantat-Element (**12**) und einer, mit der ersten Führungsfläche zusammenwirkenden, zweiten Führungsfläche am distalen Implantat-Element **(14).**

15. Implantat-Anordnung **(10)** nach Anspruch **1,**
**dadurch gekennzeichnet,**
**dass** an ihr eine integrale Kronenaufnahme **[12.4]** zur Aufnahme einer Krone (**50**) gebildet ist.

16. Implantat-Anordnung **(10)** nach Anspruch **15**,
**dadurch gekennzeichnet,**
**dass** die Kronenaufnahme (**12.4**) am distalen Endbereich des proximalen Implantat-Elementes (**12**) gebildet ist.

17. Implantat-Anordnung **(10)** nach Anspruch **16**,
**dadurch gekennzeichnet,**
**dass** das proximale Implantat-Element (**12**) und das distale Implantat-Element (**14**) mittels eines Spanngliedes, beispielsweise mittels der Krone (**50**), zusammenspannbar sind.

18. Implantat-Anordnung **(10)** nach Anspruch **5**,
**dadurch gekennzeichnet,**
**dass** die temporären Bestandteile (**16, 18**) der Implantat-Anordnung (**10**) so ausgebildet und an den definitiven Bestandteilen (**12, 14**) der Implantat-Anordnung (**10**) befestigt sind, dass sie nach der Distraktion des distalen Kieferknochenbereiches (**44**) mit Hilfe von Ultraschall von den definitiven Bestandteilen (**12, 14**) lösbar sind.

19. Implantat-Anordnung **(10)** nach einem der Ansprüche **[1** bis **17] 1** bis **18**,
**dadurch gekennzeichnet,**
**dass** das proximale Implantat-Element (**12**) und/oder das distale Implantat-Element (**14**) einen Gewindeabschnitt (**12.1**) beziehungsweise (**14.2**) mit einem Aussengewinde aufweist, zur Befestigung im Kieferknochen (**40.1**), wobei das Aussengewinde vorzugsweise ein selbstschneidendes Gewinde ist.

20. Krone (**50**), mit einer Implantat-Anordnung (**10**) nach einem der Ansprüche **1** bis **19**.

## Claims

1. Implant arrangement **(10)** for a dental prosthesis **(1)** designed to be implanted in a jawbone **(40.1)** in the direction of a longitudinal axis **(A)** of an implant, this implant arrangement **(10)** displaying:
- a proximal implant element **(12)** with an extension **(12.0)** extending in the distal direction and an outer implant thread section **(12.1)** designated to be implanted in a proximal jawbone zone **(42)** of the jawbone **(40.1),** and
- a distal implant element **(14)** with a cylindrical recess **(14.1),** a first, proximal, outer implant thread section **(14.2)** and second, distal implant thread section **(14.4)** designated to be implanted in a distal jawbone zone **(44)** of the jawbone **(40.1)** by a screwing motion, the distal implant element **(14)** being designed to be rotated and displaced coaxially on the protruding extension **(12.0)** of the proximal implant element **(12),**
these implant elements (**12, 14**) being made for the purpose of distraction of the distal jawbone zone (**44**) relative to the proximal jawbone zone (**42**) in the direction of the longitudinal axis (**A**) of the implant,
**characterized in that**
the second, distal thread section (**14.4**) of the distal implant element (**14**) possesses an outer thread having a smaller external diameter than the external diameter of the first, proximal outer thread section (**14.2**), so that the distal implant element (**14**) can be screwed as far as possible into the distal jawbone zone (**44**) until abutting against the proximal implant element **(12).**

2. Implant arrangement (**10**) according to claim **1**,
**characterized in that**
the distal implant element (**14**) that can be screwed into the distal jawbone zone (**44**), after abutting against the proximal implant element (**12**) can be rotated further about the longitudinal axis (**A**) and the distal jawbone zone (**44**) can thereby be displaced in the distal direction relative to the proximal jawbone zone (**42**) in order to effect the distraction and to dislodge the distal jawbone zone (**44**) from the proximal jawbone zone **(42)** with a fissure **(43)** between them.

3. Implant arrangement **(10)** according to any one of Claims **1** to **2**,
**characterized in that**
the proximal implant element **(12)** and the distal implant element **(14)** are definitive components of the implant arrangement **(10)** and are designated remain fixed in the jawbone **(40.1)** after assembly of the dental prosthesis **(1).**

4. Implant arrangement **(10)** according to any one of Claims **1** to **3**,
**characterized in that**
it displays means (**16, 18**) to rotate the distal implant element **(14),** preferably after at least partial dislodgment of the distal jawbone zone **(44)** from the proximal jawbone zone **(42),** in order to distract the distal jawbone zone **(44)** relative to the proximal jawbone zone **(42).**

5. Implant arrangement **(10)** according to Claim **4,**
**characterized in that**
at least some of the means (**16, 18**) for the distraction of the distal jawbone zone **(44)** constitute temporary components of the implant arrangement **(10)** and are designated to be removed from the definitive elements (**12, 14**) of the implant arrangement **(10)** after distraction of the distal jawbone zone **(44).**

6. Implant arrangement **(10)** according to either of Claims **4** to **5**,
**characterized in that**
the means for dislodging the distal jawbone zone **(44)** display an auxiliary element **(16)** which is designated to remain solidly united with the distal implant element **(14)** during distraction of the distal jawbone zone **(44).**

7. Implant arrangement **(10)** according to Claim **6**,
**characterized in that**
the means for displacing the distal jawbone zone **(44)** display an additional auxiliary element **(18)** which is movable relative to the auxiliary element **(16)** along the direction of the longitudinal axis **(A)** of the implant, the additional auxiliary element **(16)** being desigated to remain, during the distraction of the distal jawbone zone **(44),** solidly united with the implant element **(12)** during movement.

8. Implant arrangement **(10)** according to Claim **6** or **7,**
**characterized in that**
the auxiliary element **(16)** is designed to be a tension member and can be adjusted in order to effect distraction of the distal jawbone zone **(44).**

9. Implant arrangement **(10)** according to Claim **7** or **8**,
**characterized in that**
the additional auxiliary element **(18)** takes the form of a positioning device and can be adjusted for the purpose of distraction of the distal jawbone zone **(44).**

10. Implant arrangement **(10)** according to any one of Claims **6** to **9**,
**characterized in that**
the auxiliary element **(16)** and, where applicable, the additional auxiliary element **(18),** display markings to show their adjustment rate.

11. Implant arrangement **(10)** according to Claim **4**,
**characterized in that**
the proximal implant element **(12),** the distal implant element **(14)** and the means (**16, 18, 19**) for distraction of the distal implant element **(14)** are so constructed that the implant arrangement **(10)** can be fitted forming a seal in the jawbone **(40.1)** and in a zone of gum tissue **(40.2)** contiguous with the jawbone **(40.1).**

12. Implant arrangement **(10)** according to Claim **7**,
**characterized in that**
the means (**16, 18**) for distraction of the distal jawbone zone **(44)** comprise an auxiliary element **(19)** acting as a sealing element, preferably arranged between the proximal implant element **(12)** and the additional auxiliary element **(18).**

13. Implant arrangement **(10)** according to Claim **6**,
**characterized in that**
the auxiliary element **(16)** possesses a sealing surface, preferably tapered, with which, once fitted, it can make close contact with a complementary sealing surface of the implant arrangement **(10)** in order to form a seal.

14. Implant arrangement **(10)** according to Claim **1**,
**characterized in that**
it comprises a guide device, with a first guiding surface on the proximal implant element **(12)** and a second guiding surface that interacts with the first guiding surface on the distal implant element **(14).**

15. Implant arrangement **(10)** according to Claim **1**,
**characterized in that**
an integral crown support **(12.4)** is formed for supporting a crown **(50).**

16. Implant arrangement **(10)** according to Claim **15**,
**characterized in that**
the crown support **(12.4)** is formed on the distal end zone of the proximal implant element **(12).**

17. Implant arrangement **(10)** according to Claim **16**,
**characterized in that**
the proximal implant element **(12)** and the distal implant element **(14)** can be clamped together by means of a clamping membernt, for example by means of the crown **(50).**

18. Implant arrangement **(10)** according to Claim **5**,
**characterized in that**
the temporary components (**16, 18**) of the implant arrangement **(10)** are so constructed and fixed to the definitive components **(12, 14)** of the implant arrangement **(10)** that after distraction of the distal jawbone zone **(44)** they can be separated from the definitive components (**12**, **14**) by means of ultrasound.

19. Implant arrangement **(10)** according to any one of Claims **1** to **18**,
**characterized in that**
the proximal implant element **(12)** and/or the distal implant element **(14)** displays a thread section **(12.1)** or **(14.2)** respectively with an external thread for fixing it into the jawbone **(40.1),** this external thread being preferably a self-tapping thread.

20. Crown **(50)** with an implant arrangement **(10)** according to any one of Claims **1** to **19**.

## Revendications

1. Ensemble d'implant **(10)** pour prothèse dentaire **(1),** défini de façon à être implanté, dans la direction d'un axe longitudinal d'implant **(A),** dans un os de mâchoire **(40.1),** dans lequel l'ensemble d'implant **(10)** comporte
- un élément d'implant proximal **(12)** comportant une tubulure **(12.0)** qui s'étend dans la direction distale et un segment d'implant fileté externe **(12.1)** qui est défini de façon à être implanté dans une zone proximale d'os de mâchoire (42) de l'os de mâchoire **(40.1),** et
- un élément d'implant distal **(14)** comportant un évidement cylindrique **(14.1),** un premier segment d'implant fileté externe proximal **(14.2)** et un second segment fileté distal **(14.4),** défini de façon à être implanté dans une zone distale d'os de mâchoire **(44)** de l'os de mâchoire **(40.1)** par un mouvement de vissage, l'élément d'implant distal **(14)** pouvant être pivoté et déplacé de façon co-axiale sur la tubulure allongée **(12.0)** de l'élément d'implant proximal **(12),**
les éléments d'implant **(12,14)** étant conçus pour une distraction de la zone distale de l'os de mâchoire **(44)** par rapport à la zone proximale de l'os de mâchoire **(42)** dans la direction de l'axe longitudinal de l'implant **(A),**
**caractérisé**
**en ce que** le second segment fileté distal (14.4) de l'élément d'implant distal **(14)** possède un filetage externe dont le diamètre externe est plus petit que le diamètre externe du premier segment fileté externe proximal **(14.2),** si bien que l'élément d'implant distal (14) peut être vissé aussi profondément que possible dans la zone distale de l'os de mâchoire **(44)** jusqu'à buter au niveau de l'élément d'implant proximal **(12).**

2. Ensemble d'implant **(10)** selon la revendication **1**,
**caractérisé**
**en ce que** l'élément d'implant distal (14) pouvant être vissé dans la zone distale de l'os de mâchoire (44), après la butée au niveau de l'élément d'implant proximal **(12)** peut être encore tourné autour de l'axe longitudinal de l'implant **(A)** et à cet égard la zone distale de l'os de mâchoire **(44)** peut être déplacée par rapport à la zone proximale de l'os de mâchoire **(42)** dans une direction distale afin de réaliser la distraction et d'écarter la zone distale de l'os de mâchoire **(44)** de la zone proximale de l'os de mâchoire **(42)** d'une fente **(43).**

3. Ensemble d'implant **(10)** selon l'une des revendications **1** à **2**,
**caractérisé**
**en ce que** l'élément d'implant proximal **(12)** et l'élément d'implant distal **(14)** sont des éléments définitifs de l'ensemble d'implant **(10)** et sont destinés à rester fixés dans l'os de mâchoire **(40.1)** après le montage de la prothèse dentaire **(1).**

4. Ensemble d'implant **(10)** selon l'une des revendications **1** à **3**,
**caractérisé**
**en ce qu'**il comprend des moyens (**16, 18**) permettant, de préférence après une séparation au moins partielle de la zone distale de l'os de mâchoire **(44)** de la zone proximale de l'os de mâchoire **(42),** de faire tourner l'élément d'implant distal **(14)** afin de distraire la zone distale de l'os de mâchoire **(44)** par rapport à la zone proximale de l'os de mâchoire **(42).**

5. Ensemble d'implant **(10)** selon la revendication **4,**
**caractérisé**
**en ce qu'**au moins une partie des moyens **(16,18)** conçus pour distraire la zone distale de l'os de mâchoire **(44)** sont des éléments temporaires de l'ensemble d'implant **(10)** qui sont destinés de façon à être séparés des éléments définitifs **(12,14)** de l'ensemble d'implant **(10)** après la distraction de la zone distale de l'os de mâchoire **(44).**

6. Ensemble d'implant **(10)** selon l'une des revendications **4** à **5**,
**caractérisé**
**en ce que** les moyens définis pour déplacer la zone distale de l'os de mâchoire **(44)** présentent un élément auxiliaire **(16)** qui permet, pendant la distraction de la zone distale de l'os de mâchoire **(44),** d'être solidaire de l'élément d'implant distal **(14)** selon les déplacements.

7. Ensemble d'implant **(10)** selon la revendication **6**,
**caractérisé**
**en ce que** les moyens définis de façon à déplacer la zone distale de l'os de mâchoire **(44)** présentent un autre élément auxiliaire **(18)** qui est mobile par rapport à l'élément auxiliaire **(16)** dans la direction de l'axe longitudinal d'implant **(A),** l'autre élément auxiliaire **(16)** étant conçu pour être solidaire avec l'élément d'implant proximal **(12)** selon les mouvements pendant la distraction de la zone distale de l'os de mâchoire **(44).**

8. Ensemble d'implant **(10)** selon la revendication **6** ou **7,**
**caractérisé**
**en ce que** l'élément auxiliaire **(16)** est exécuté en tant qu'organe de traction et en ce qu'il est réglable à des fins de distraction de la zone distale de l'os de mâchoire **(44).**

9. Ensemble d'implant **(10)** selon la revendication **7** ou **8**,
**caractérisé**
**en ce que** l'autre élément auxiliaire **(18)** est exécuté en tant qu'organe de réglage, qui peut être réglé à des fins de distraction de la zone distale de l'os de mâchoire **(44).**

10. Ensemble d'implant selon l'une des revendications **6** à **9**,
**caractérisé**
**en ce que** l'élément auxiliaire **(16)** et éventuellement l'autre élément auxiliaire **(18)** présentent des marquages pour indiquer l'étendue de leur réglage.

11. Ensemble d'implant **(10)** selon la revendication **4**,
**caractérisé**
**en ce que** l'élément d'implant proximal **(12),** l'élément d'implant distal **(14)** et les moyens **(16, 18,19)** de distraction de l'élément d'implant distal **(14)** sont exécutés de façon telle que l'ensemble d'implant **(10)** peut être monté de façon étanche dans l'os de mâchoire **(40.1)** et dans une zone de la gencive **(40.2)** contiguë à l'os de mâchoire **(40.1).**

12. Ensemble d'implant **(10)** selon la revendication **7**,
**caractérisé**
**en ce que** les moyens (**16, 18**) permettant de distraire la zone distale de l'os de mâchoire **(44)** comprennent un élément auxiliaire **(19)** agissant en tant qu'élément d'étanchéité, qui est de préférence disposé entre l'élément d'implant proximal **(12)** et l'autre élément auxiliaire **(18).**

13. Ensemble d'implant (10) selon la revendication **6**,
**caractérisé**
**en ce que** l'élément auxiliaire **(16)** possède une surface d'étanchéité de préférence approchante, avec laquelle il repose de façon étanche à l'état monté contre une surface d'étanchéité complémentaire de l'ensemble d'implant **(10).**

14. Ensemble d'implant **(10)** selon la revendication **1**,
**caractérisé**
**en ce qu'**il possède un dispositif de guidage comportant une première surface de guidage au niveau de l'élément d'implant proximal **(12)** et une seconde surface de guidage, coopérant avec la première surface de guidage, au niveau de l'élément d'implant distal **(14).**

15. Ensemble d'implant **(10)** selon la revendication **1**,
**caractérisé**
**en ce qu'**est formé à son niveau un logement de couronne intégrale **(12.4)** destiné à recevoir une couronne **(50).**

16. Ensemble d'implant **(10)** selon la revendication **15**,
**caractérisé**
**en ce que** le logement de couronne **(12.4)** est formé au niveau de la zone d'extrémité distale de l'élément d'implant proximal **(12).**

17. Ensemble d'implant **(10)** selon la revendication **16**,
**caractérisé**
**en ce que** l'élément d'implant proximal **(12)** et l'élément d'implant distal **(14)** peuvent être serrés au moyen d'un organe de précontrainte, par exemple au moyen de la couronne **(50).**

18. Ensemble d'implant **(10)** selon la revendication **5**,
**caractérisé**
**en ce que** les éléments temporaires (**16, 18**) de l'ensemble d'implant **(10)** sont conçus et fixés au niveau des éléments définitifs (**12, 14**) de l'ensemble d'implant **(10)** de façon telle qu'après la distraction de la zone distale de l'os de mâchoire **(44),** ils peuvent être détachés des éléments définitifs **(12,14)** à l'aide d'ultrasons.

19. Ensemble d'implant **(10)** selon l'une des revendications **1** à **18**,
**caractérisé**
**en ce que** l'élément d'implant proximal **(12)** et/ou l'élément d'implant distal **(14)** présente un segment fileté **(12.1)** respectivement **(14.2)** comportant un filetage externe, destiné à la fixation dans l'os de mâchoire **(40.1),** le filetage externe étant de préférence un filet auto-taraudeur.

20. Couronne **(50)** ayant un ensemble d'implant **(10)** selon l'une des revendications **1** à **19**.
